(19) **Europäisches Patentamt**

**European Patent Office**

**Office européen des brevets**

(11) **EP 1 035 239 B1**

(12) **EUROPEAN PATENT SPECIFICATION**

(45) Date of publication and mention
of the grant of the patent:
**11.05.2005 Bulletin 2005/19**

(51) Int Cl.⁷: **D01F 9/00**, A61L 15/00,
A61L 15/28

(21) Application number: **00104794.3**

(22) Date of filing: **06.03.2000**

(54) **Absorbent, flexible, structure comprising starch fibers**

Absorbierende und flexible Struktur mit Stärkefasern

Structure absorbente et flexible comprenant des fibres d' amidon

(84) Designated Contracting States:
**AT BE CH CY DE DK ES FI FR GB GR IE IT LI LU
MC NL PT SE**

(30) Priority: **08.03.1999 US 264401**

(43) Date of publication of application:
**13.09.2000 Bulletin 2000/37**

(73) Proprietor: **THE PROCTER & GAMBLE COMPANY
Cincinnati, Ohio 45202 (US)**

(72) Inventors:
• **The inventors have agreed to waive their
entitlement to designation.**

(74) Representative: **Hirsch, Uwe Thomas et al
Procter & Gamble Service GmbH
Sulzbacher Strasse 40-50
65824 Schwalbach am Taunus (DE)**

(56) References cited:
**GB-A- 1 247 474          US-A- 5 516 815**

• **PATENT ABSTRACTS OF JAPAN vol. 1998, no.
05, 30 April 1998 (1998-04-30) & JP 10 008364 A
(CHISSO CORP), 13 January 1998 (1998-01-13)**
• **PATENT ABSTRACTS OF JAPAN vol. 011, no.
206 (C-433), 3 July 1987 (1987-07-03) & JP 62
028410 A (CHISSO CORP), 6 February 1987
(1987-02-06)**
• **DATABASE WPI Week 19982 Derwent
Publications Ltd., London, GB; AN 1998-012572
XP002149061 "Absorption article - uses
nonwoven fibre aggregate comprising water
disintegrable fibre and thermoplastic fibre" & JP
09 276331 A (CHISSO CORP), 28 January 1997
(1997-01-28)**

**Description**

FIELD OF THE INVENTION

The present invention relates to pseudo-thermoplastic starch extruded in the form of fibers. Starch may be extruded and either meltblown or spunbonded to form fibrous low density structures.

BACKGROUND OF THE INVENTION

It is well recognized that starch molecules come in two forms: the substantially linear amylose polymer and the highly branched amylopectin polymer. These two forms of starch have very different properties, probably due to the ease of association of the hydroxyl groups among different molecules. The molecular structure of amylose is essentially linear with two to five relatively long branches. The average degree of polymerization of the branches is about 350 monomer units. Under conditions that provide sufficient freedom of molecular movements, primarily by dilution with suitable solvents, and in some instances, dilution coupled with heating, the linear amylose chains can be oriented into preferentially parallel alignments such that the hydroxyl groups on one chain are in close proximity with those on the adjacent chains. The alignment of neighboring amylose molecules is believed to facilitate intermolecular hydrogen bonding. Consequently the amylose molecules form strong aggregates. In contrast, the molecular structure of amylopectin is highly branched via 1,6-$\alpha$ linkages. The average degree of polymerization of the branches is about 25 monomer units. Due to the highly branched structure, the amylopectin molecules can not move as freely and do not align and associate as readily.

Attempts have been made to process natural starch on standard equipment and existing technology known in the plastic industry. Since natural starch generally has a granular structure, it needs to be "destructurized" and/or modified before it can be melt processed like a thermoplastic material. For destructurization, the starch is typically heated above its softening and melting temperature under a pressurized condition. Melting and disordering of the molecular structure of the starch granule takes place and a destructurized starch is obtained. Chemical or enzymatic agents may also be used to destructurize, oxidize, or derivatize the starch. Modified starches have been used to make biodegradable plastics, wherein the modified starch is blended as an additive or the minor component with petroleum-based or synthetic polymers. However, when the modified starch is processed by itself or as the major component in a blend with other materials using conventional thermoplastic processing techniques, such as molding or extrusion, the finished parts tend to have a high incidence of defects. Moreover, the modified starch (alone or as the major component of a blend) has been found to have poor melt extensibility; consequently, it cannot be successfully processed by uniaxial or biaxial extensional processes into fibers, films, foams or the like.

Previous attempts to produce starch fibers relate principally to wet-spinning processes. For Example, a starch/solvent colloidal suspension can be extruded from a spinneret into a coagulating bath. This process relies on the marked tendency of amylose to align and form strongly associated aggregates to provide strength and integrity to the final fiber. Any amylopectin present is tolerated as an impurity that adversely affects the fiber spinning process and the strength of the final product. Since it is well known that natural starch is rich in amylopectin, earlier approaches include pre-treating the natural starch to obtain the amylose-rich portion desirable for fiber spinning. Clearly this approach is not economically feasible on a commercial scale since a large portion (i.e, the amylopectin portion) of the starch is discarded. In more recent developments, natural starch, typically high in natural amylopectin content, can be wet-spun into fibers. However, the wet-spun fibers are coarse, typically having fiber diameters greater than 50 microns. Additionally, the large quantity of solvent used in this process requires an additional drying step and a recovery or treatment step of the effluent. Some references for wet-spinning starch fibers include U.S. Patent No. 4,139,699 issued to Hernandez et al. on February 13, 1979; U.S. Patent No. 4,853,168 issued to Eden et al. on August 1, 1989; and U.S. Patent No. 4,234,480 issued to Hernandez et al. on January 6, 1981.

U.S. Patent Nos. 5,516,815 and 5,316,578 to Buehler et al. relate to starch compositions for making starch fibers from a melt spinning process. The melt starch composition is extruded through a spinnerette to produce filaments having diameters slightly enlarged relative to the diameter of the die orifices on the spinnerette (i.e., a die swell effect). The filaments are subsequently drawn down mechanically or thermomechanically by a drawing unit to reduce the fiber diameter. The major disadvantage of the starch composition of Buehler et al. is that it does not use high molecular weight polymers, which enhance the melt extensibility of starch compositions. Consequently, the starch composition of Buehler et al. could not be successfully melt attenuated to produce fine fibers of 25 microns or less in diameter.

Other thermoplastically processable starch compositions are disclosed in U.S. Patent No. 4,900,361, issued on August 8,1989 to Sachetto et al.; U.S. Patent No. 5,095,054, issued on March 10, 1992 to Lay et al.; U.S. Patent No. 5,736,586, issued on April 7, 1998 to Bastioli et al.; and PCT publication WO 98/40434 filed by Hanna et al. published March 14, 1997. These starch compositions do not contain the high molecular weight polymers that are necessary to achieve the desired melt viscosity and melt extensibility, which are critical material characteristics to producing fine fibers, thin films or thin-walled foams.

Cellulose fibrous webs such as paper are well known in the art. Low density fibrous webs are in common use today in products such as paper towels, toilet tissue, facial tissue, napkins, wet wipes, and the like. The large demand for such paper products has created a need for improvements in the products and in the methods of their manufacture.

There are several well known concerns regarding the papermaking industry requiring papermaking manufacturers to balance the costs of machinery and resources with the total cost of delivering the paper products to the consumers. First instance, the popularity of paper products has created an increasing demand on wood based cellulosic fibers resulting in a rapid depletion of trees due to deforestation.

In addition, during conventional papermaking operations wood cellulosic fibers are repulped, beaten or refined to achieve a level of fiber hydration in order to form an aqueous pulp slurry. Processes for the making of paper products for use in tissue, toweling, and sanitary products generally involve the preparation of the aqueous slurry and then subsequently removing the water from the slurry while contemporaneously rearranging the fibers therein to form a paper web. Subsequent to dewatering, the web is processed into a dry roll or sheet form and eventually converted into a consumer package. Various types of machinery must be employed to assist in the dewatering process and converting operations requiring a significant investment in capital.

Further, the conventional papermaking operation involves the incorporation of additives into the pulp in order to achieve specific end properties. For instance, additives such as strength resins, debonding surfactants, softening agents, pigments, lattices, synthetic microspheres, fire-retardants, dyes, perfumes, etc., are often employed in the manufacture of paper. The efficient retention of these additives at the wet end of a papermaking process presents difficulty to the manufacturer since that portion which is not retained creates not only an economic loss but also significant pollution problems if it becomes part of a plant effluent. Additives can also be added to the paper web subsequent to dewatering via coating or saturation processes commonly known in the art. These processes usually require that excess heating energy be consumed to redry the paper after coating. Moreover, in some instances, the coating systems are required to be solvent based which increases capital costs and requires recovery of volatile materials to meet regulatory requirements.

Various natural fibers other than cellulose as well as a variety of synthetic fibers have been employed in making paper. However, these replacements have failed to provide a commercially acceptable substitute for cellulose due to their high cost, poor bonding properties, chemical incompatibilities, and handling difficulties in papermaking systems. Starch fibers have been suggested as a substitute for cellulose in various aspects of the papermaking process, however, commercial attempts to use such fibers have been unsuccessful. As a result, paper products are still being manufactured almost exclusively from wood base cellulosic ingredients.

Starch is a plant carbohydrate having a structure analogous to cellulose. Whereas cellulose is a polymer of D-glucan monomers connected via 1,4-β linkages, starch is a polymer of D-glucose monomers connected via primarily 1,4-α linkages. Because starch is available via agricultural resources, it is cheap and plentiful. While starch has been incorporated into various aspects of the papermaking process, attempts to use starch fibers as cellulose fiber replacement in commercial papermaking processes have not been successful.

Consequently, there is a need for an inexpensive and melt processable composition from natural starches. Such a melt processable starch composition should not require evaporation of a large quantity of solvents or produce a large amount of effluent during the processing operation. Moreover, such a starch composition should have melt rheological properties suitable for use in conventional plastic processing equipment

There is also a need for a starch composition suitable for use in uniaxial or biaxial extensional processes to produce fibers, films, sheets, foams, shaped articles, and the like economically and efficiently. Specifically, the starch composition should have melt rheological properties suitable for uniaxially or biaxially extensional processes in its melt phase in a substantially continuous manner, i.e., without excessive amount of melt fracture or other defects.

Further, there is a need for a low density flexible structure comprising starch fibers utilizing extrusion and fiber spinning techniques. Particularly, a low density flexible structure comprising starch fibers, wherein the structure has improved tensile strength, softness, and absorbency properties relative to cellulosic pulp fiber structures and the like, while maintaining biodegradability and flushability.

SUMMARY OF THE INVENTION

The present invention provides an absorbent flexible structure comprising starch fibers. Naturally occurring starch in the presence of water, plasticizers and other additives is melt extruded and spun into fibers to form an absorbent flexible structure having an apparent density ranging from 0.02 $g/cm^3$ to 0.20 $g/cm^3$ and a basis weight ranging from 10 $g/m^2$ to 450 $g/m^2$.

The starch fibers making up the structure can have a size ranging from about 0.01 decitex to about 135 decitex. In a preferred embodiment the fibers can have a size ranging from about 0.02 decitex to about 30 decitex, and most preferably ranging from about 0.02 to about 5 decitex. In addition, fibers making up the structure of the present invention can have a glass transition temperature ranging from about -30 °C to about 150 °C, more preferably from about -30 °C to about 100 °C, and most preferably from about -30 °C to about 25 °C.

Exemplary physical properties of the flexible structure of the present invention include dry tensile strength and wet tensile strength. The dry tensile strength of the structure, which is measured as a geometric mean tensile strength, can range from about 10 g/cm to about 1200 g/cm, more preferably from about 30 g/cm to about 600 g/cm, and most preferably from about 40 g/cm to about 475 g/cm. The wet tensile strength of the structure, which is also measured as a geometric mean tensile strength, can range from about 2 g/cm to about 400 g/cm, and more preferably from about 2 g/cm to about 200 g/cm.

BRIEF DESCRIPTION OF THE DRAWINGS

These and other features, aspects and advantages of the present invention will become better understood with regard to the following description, appended claims, and accompanying drawings where:

Figure 1a illustrates a torque rheometer assembly used to produce starch fibers.
Figure 1b illustrates the twin screw elements attached to the drive unit and disposed within the barrel of the torque rheometer assembly illustrated in Figure 1a.
Figure 1c shows a torque rheometer assembly having a melt blowing die used to produce fine starch fibers of the present invention.
Figure 2a illustrates a vented twin screw extruder assembly.
Figure 2b illustrates the screw and mixing element configuration for the extrusion assembly depicted in Figure 2a.
Figure 3a illustrates a non-vented twin screw extruder assembly.
Figure 3b illustrates the screw and mixing element configuration for the extrusion assembly depicted in Figure 3a.
Figure 4 illustrates a spinneret and a drawing unit used for pseudo-thermoplastic starch melt fiber spinning.
Figure 5 illustrates the sample rack and cover used for determining absorbency of the starch fiber structures.
Figure 6 illustrates the cross section of the frames for the sample rack and cover illustrated in Figure 5.
Figure 7a is the Scanning Electron Micrographs of fine starch fibers of the present invention shown on a 200 micron scale.
Figure 7b is the Scanning Electron Micrographs of fine starch fibers of the present invention shown on a 20 micron scale.

DETAILED DESCRIPTION OF THE INVENTION

Definitions

As used herein, the following terms have the following meanings:

Pseudo-thermoplastic composition is intended to denote materials which by the influence of elevated temperatures may be softened to such a degree that they can be brought into a flowable state, and in this condition may be shaped as desired. Pseudo-thermoplastic materials may be formed under simultaneous influence of heat and pressure. Pseudo-thermoplastic compositions differ from thermoplastic compositions in that the softening or liquefying of the pseudo-thermoplastic is caused by softeners or solvents present without which it would be impossible to bring them by any temperature or pressure into a soft or flowable condition necessary for shaping since pseudo thermoplastics do not melt as such. The influence of water content on the glass transition temperature and melting temperature of starch can be measured by differential scanning calorimetery as described by Zeleznak and Hoseny "Cereal Chemistry", Vol. 64, No. 2, pp. 121-124, 1987.

Pseudo-thermoplastic melt is a pseudo-thermoplastic material in a flowable state.

Glass transition temperature, $T_g$, is the temperature at which the material changes from a viscous or rubbery condition to a hard and relatively brittle condition.

Basis weight is the weight (in grams) per unit area (in square meters) of a sample reported in grams per square meter.

Caliper is the macroscopic thickness of a sample measured as described below.

Apparent density is the basis weight of the sample divided by the caliper with appropriate unit conversions incorporated therein. Apparent density used herein has the units of grams / centimeters cubed (g/cm$^3$).

Machine direction, designated MD, is the direction parallel to the flow of the starch fiber structure through the product manufacturing equipment.

Cross machine direction, designated CD, is the direction perpendicular to the machine direction in the same plane of the starch fiber structure.

Geometric Mean Dry Tensile Strength (GMDT) is the square root of the product of the machine and cross-machine dry tensile strengths (in grams/cm). The value of GMDT is reported in grams/cm.

Geometric mean wet tensile strength (GMWT) is the square root of the product of the machine and cross-machine wet tensile strengths (in grams/cm). The value of GMWT is reported in grams/cm.

Structure is an arrangement of one or more parts forming a substance or body.

Absorbency is the ability of a material to take up fluids by various means including capillary, osmotic, solvent or chemical action and retain such fluids.

Flexibility indicates the capability of being deformed under a given load without being broken and with or without returning of itself to its former shape.

A fiber is a slender object having a major axis which is very long compared to the two orthogonal axes and having an aspect ratio of at least 4/1, preferably at least 10/1.

Decitex ,dtex, is a unit of measure for a fiber expressed in $\frac{grams}{10,000\ meters}$

Flushability is determined by the geometric mean decayed wet tensile strength (GMDWT) (defined below). A flushable structure has a geometric mean decayed wet tensile of less than about 20 g/cm and more preferably less than about 10 g/cm.

The term "bound water" means the water found naturally occurring in starch and before mixing of starch with other components to make the composition of the present invention. The term "free water" means the water that is added in making the composition of the present invention. A person of ordinary skill in the art would recognize that once the components are mixed in a composition, water can no longer be distinguished by its origin.

All percentages, ratios and proportions used herein are by weight percent of the composition, unless otherwise specified.

The specification contains a detailed description of (1) exemplary materials of the present invention, (2) exemplary processes for producing the present invention, (3) material properties of the present invention, and (4) analytical procedures for measuring properties of the present invention

(1) Exemplary Materials

For the present invention a starch polymer is mixed with water, plasticizers and other additives and melt extruded to produce fibers . Standard meltblowing or spunbonding techniques are used to produce starch fiber structures. Such structures may be absorbent and flexible. These structures may be used as substitutes for paper products such as paper towels, napkins, toilet tissues, facial tissues, place mats and wet wipes. Other uses include, but are not limited to, oil absorbents, seed carriers, fillers for concrete, pressed board, and other construction materials, time released watering aids for house plants, and thin films.

The starch fibers of the present invention may be useful for forming fibrous structures and also forming absorbent materials, as described above. The absorbent structures/fibrous materials comprising the starch fibers of the present invention may have from a trace amount to one hundred percent (100%) starch fibers, or a blend of starch fibers and other suitable fibers. Other suitable fibers for the blend include cellulose fibers, synthetic fibers, and a combination thereof.

Starch polymers can include any naturally occurring (unmodified) starch, physically modified starch, chemically modified starch, biologically modified starch or combinations thereof.

Naturally occurring starch is generally a mixture of linear amylose and branched amylopectin polymer of D-glucose units. The amylose is a substantially linear polymer of D-glucose units joined by $(1,4)$-$\alpha$-D links. The amylopectin is a highly branched polymer of D-glucose units joined by $(1,4)$-$\alpha$-D links and $(1,6)$-$\alpha$-D links at the branch points. Naturally occurring starch typically contains relatively high amylopectin, for example, corn starch (64-80% amylopectin), waxy maize (93-100% amylopectin), rice (83-84% amylopectin), potato (about 78% amylopectin), and wheat (73-83% amylopectin). Though all starches are useful herein, the present invention is most commonly practiced with high amylopectin natural starches derived from agricultural sources, which offer the advantages of being abundant in supply, easily replenishable and inexpensive in price.

Suitable naturally occurring starches can include, but are not limited to, corn starch, potato starch, sweet potato starch, wheat starch, sago palm starch, tapioca starch, rice starch, soybean starch, arrow root starch, bracken starch, lotus starch, waxy maize starch, high amylose corn starch, and commercial amylose powder. Naturally occurring starches particularly, corn starch and wheat starch, are the preferred starch polymers of choice due to their economy and availability.

Physically modified starch is formed by changing the dimensional structure. Physical modifications of the starch may be intramolecular or intermolecular modifications. Intramolecular modifications include reduced molecular weight and/or molecular weight distribution, changes in the polymer chain conformation, and the like. Intermolecular modifications include melting and/or disordering the starch molecules, reduction in crystallinity, crystallite size, and granular size, and the like. These physical modifications may be achieved by input of energy (such as thermal, mechanical, thermomechanical, electromagnatic, ultrasonic, and the like), pressure, moisture, fractionation, and combinations thereof. Physically modified starch can include $\alpha$ starch, fractionated starch, moisture and heat treated starch.

Chemical modifications of starch typically include acid or alkali hydrolysis and oxidative chain scission to reduce molecular weight and molecular weight distribution. Suitable compounds for chemical modification of starch include organic acid such as citric acid, acetic acid, glycolic acid, and adipic acid; inorganic acids such as hydrochloric acid, sulfuric acid, nitric acid, phosphoric acid boric acid, and partial salts of polybasic acids, e.g., $KH_2PO_4$, $NaHSO_4$; group Ia or IIa metal hydroxides such as sodium hydroxide, potassium hydroxide; ammonia; oxidizing agents such as hydrogen peroxide, benzoyl peroxide, ammonium persulfate, potassium permagnate, sodium bicarbonate, hypochloric salts, and the like; and mixtures thereof. Preferred chemical agents or the present invention include ammonium persulfate, sulfuric acid, hydrochloric acid, and mixtures thereof.

Chemically modified starch may be formed by reaction of its OH groups with alkylene oxides, and other ether-, ester-, urethane-, carbamate-, or isocyanate- forming substances. Hydroxyalkyl, acetyl, or carbamate starches or mixtures thereof are preferred chemically modified starches. The degree of substitution of the chemically modified starch is 0.05 to 3.0, preferably 0.05 to 0.2.

Biological modifications of starch include bacterial digestion of the carbohydrate bonds, or enzymatic hydrolysis using enzymes such as amylase, amylopectase, and the like.

The starch desirably has a bound water content of about 5% to 16% by weight of starch. A water content of 8% to 12% by weight of starch is particularly preferred. The amylose content of the starch is 0% to 80% by weight of starch, preferably 20% to 30% by weight of starch.

Natural, unmodified starch generally has a very high average molecular weight and a broad molecular weight distribution (e.g. natural corn starch has an average molecular weight of about 60,000,000 and a molecular weight distribution greater than 1000). The average molecular weight of starch can be reduced to the desirable range for the present invention by chain scission (oxidative or enzymatic), hydrolysis (acid or alkaline catalyzed), physical/mechanical degradation (e.g., via the thermomechanical energy input of the processing equipment), or combinations thereof. These reactions also reduce the molecular weight distribution of starch to less than about 600, preferably to less than about 300. The thermomechanical method and the oxidation method offer an additional advantage, carried out in situ of the melting spinning process.

In one embodiment, the natural starch is hydrolyzed in the presence of acids, such as hydrochloric acid or sulfuric acid, to reduce the molecular weight and molecular weight distribution. In another embodiment, a chain scission agent may be incorporated into the melt spinnable starch composition such that the chain scission reaction takes place substantially concurrently with the blending of the starch with other components. Nonlimiting examples of oxidative chain scission agents suitable for use herein include ammonium persulfate, hydrogen peroxide, hypochloric salts, potassium permanganate, and mixtures thereof. Typically, the chain scission agent is added in an amount effective to reduce the weight-average molecular weight of the starch to the desirable range. For example, it is found that for uniaxial or biaxial melt attenuation processes, the starch should have a weight-average molecular weight ranging from about 1,000 to about 2,000,000, preferably from about 1,500 to about 800,000, more preferably from about 2,000 to about 500,000. It is found that compositions having modified starch in the above molecular weight range have a suitable melt shear viscosity, and thus, improved melt processability. The improved melt processability is evident in less interruptions of the process (e.g., reduced breakage, shots, defects, hang-ups) and better surface appearance and strength properties of the product.

Typically, the composition herein comprises from about 20 to about 99.99 wt%, preferably from about 30 to about 95 wt%, and more preferably from about 50 to about 85 wt%, of unmodified and/or modified starch. The weight of starch in the composition includes starch and its naturally occurring bound water content. It is known that additional free water may be incorporated as the polar solvent or plasticizer, and not included in the weight of the starch.

High molecular weight polymers (hereinafter "high polymers") which are substantially compatible with starch are also useful herein. The molecular weight of a suitable polymer should be sufficiently high to effectuate entanglements and/or associations with starch molecules. The high polymer preferably has a substantially linear chain structure. Though a linear chain having short (C1-C3) branches or a branched chain having one to three long branches are also suitable for use herein. As used herein, the term "substantially compatible" means when heated to a temperature above the softening and/or the melting temperature of the composition, the high polymer is capable of forming a substantially homogeneous mixture with the starch (i.e., the composition appears transparent or translucent to the naked eyes).

The Hildebrand solubility parameter ($\delta$) can be used to estimate the compatibility between starch and the polymer. Generally, substantial compatibility between two materials can be expected when their solubility parameters are similar. It is known that water has a $\delta_{water}$ value of 48.0 $MPa^{1/2}$, which is the highest among common solvents, probably due to the strong hydrogen bonding capacity of water. Starch typically has a $\delta_{starch}$ value similar to that of cellulose (about 344 $MPa^{1/2}$).

Without being bound by theory, it is believed that polymer suitable for use herein preferably interact with the starch molecules on the molecular level in order to form a substantially compatible mixture. The interactions range from the strong, chemical type interactions such as hydrogen bonding between polymer and starch, to merely physical entanglements between them. The polymers useful herein are preferably high molecular weight, substantially linear chain molecules. The highly branched structure of a amylopectin molecule favors the branches to interact intramolecularly, due to the proximity of the branches within a single molecule. Thus, it is believed that the amylopectin molecule has poor or ineffective entanglements/interactions with other starch molecules, particularly other amylopectin molecules. The compatibility with starch enables suitable polymers to be intimately mixed and chemically interact and/or physically entangle with the branched amylopectin molecules such that the amylopectin molecules associate with one another via the polymers. The high molecular weight of the polymer enables it to simultaneously interact/entangle with several starch molecules. That is, the high polymers function as molecular links for starch molecules. The linking function of the high polymers is particularly important for starches high in amylopectin content. The entanglements and/or associations between starch and polymers enhance the melt extensibility of the starch composition such that the composition is suitable for extensional processes. In one embodiment, it is found that the composition can be melt attenuated uniaxially to a very high draw ratio (greater than 1000).

In order to effectively form entanglements and/or associations with the starch molecules, the high polymer suitable for use herein should have a weight-average molecular weight of at least 500,000. Typically the weight average molecular weight of the polymer ranges from about 500,000 to about 25,000,000, preferably from about 800,000 to about 22,000,000, more preferably from about 1,000,000 to about 20,000,000, and most preferably from about 2,000,000 to about 15,000,000. The high molecular weight polymers are preferred due to the ability to simultaneously interact with several starch molecules, thereby increases extensional melt viscosity and reduces melt fracture.

Suitable high polymers have a $\delta_{polymer}$ such that the difference between $\delta_{starch}$ and $\delta_{polymer}$ is less than about 10 $MPa^{1/2}$, preferably less than about 5 $MPa^{1/2}$, and more preferably less than about 3 $MPa^{1/2}$. Nonlimiting examples of suitable high polymers include polyacrylamide and derivatives such as carboxyl modified polyacrylamide, acrylics and acrylic polymers and copolymers including polyacrylic acid, polymethacrylic acid, and their partial esters; vinyl polymers including polyvinyl alcohol, polyvinylacetate, polyvinylpyrrolidone, polyethylene vinyl acetate, polyethyleneimine, and the like; polyamides; polyalkylene oxides such as polyethylene oxide, polypropylene oxide, polyethylenepropylene oxide, and mixtures thereof. Copolymers made from mixtures of monomers selected from any of the aforementioned polymers are also suitable herein. Other exemplary high polymers include water soluble polysaccharides such as alginates, carrageenans, pectin and derivatives, chitin and derivatives, and the like; gums such as guar gum, xanthum gum, agar, gum arabic, karaya gum, tragacanth gum, locust bean gum, and like gums; water soluble derivatives of cellulose, such as alkylcellulose, hydroxyalkylcellulose, carboxymethylcellulose, and the like; and mixtures thereof.

Some polymers (e.g., polyacrylic acid, polymethacrylic acid) are generally not available in the high molecular weight range (i.e., 500,000 or higher). A small amount of crosslinking agents may be added to create branched polymers of suitably high molecular weight useful herein.

The high polymer is added to the composition of the present invention in an amount effective to visibly reduce the melt fracture and capillary breakage of fibers during the spinning process such that substantially continuous fibers having relatively consistent diameter can be melt spun. These polymers are typically present in the range from about 0.01 to about 10 wt%, preferably from about 0.03 to about 1 wt%, more preferably from about 0.05 to about 0.5 wt% of the composition. It is surprising to find that at a relatively low concentration, these polymers significantly improves the melt extensibility of the starch composition.

The starch compositions may optionally include additives to enhance melt flow and melt processability, particularly the extensibility of the composition under the melt processing conditions. The additives may function as plasticizers and/or diluents to reduce the melt shear viscosity of the starch composition. The plasticizers are added to the composition of the present invention in an amount effective to improve the flow, hence, the melt processability. The plasticizers may also improve the flexibility of the final products, which is believed to be due to the lowering of the glass transition temperature of the composition by the plasticizer. The plasticizers should preferably be substantially compatible with the polymeric components of the present invention so that the plasticizers may effectively modify the properties of the composition. As used herein, the term " substantially compatible" means when heated to a temperature above the softening and/or the melting temperature of the composition, the plasticizer is capable of forming a substantially homogeneous mixture with starch (i.e., the composition appears transparent or translucent to the naked eye).

A plasticizer is typically added to the starch polymer in order to lower the glass transition temperature of the starch fibers thereby enhancing the flexibility of the fibers. In addition, the presence of the plasticizer lowers the melt viscosity which in turn facilitates the melt extrusion process. The plasticizer is advantageously an organic compound having at least one hydroxyl group, preferably a polyol. Without being bound by theory, it is believed that the hydroxyl groups of the plasticizers enhance compatibility by forming hydrogen bonds with the starch matrix material. Nonlimiting examples of useful hydroxyl plasticizers include sugars such as glucose, sucrose, fructose, raffinose, maltodextrose, galactose, xylose, maltose, lactose, mannose erythrose, glycerol, and pentaerythritol; sugar alcohols such as erythritol, xylitol, malitol, mannitol and sorbitol; polyols such as ethylene glycol, propylene glycol, dipropylene glycol, butylene glycol, hexane triol, and the like, and polymers thereof; and mixtures thereof.

Also useful herein as hydroxyl plasticizers are poloxomers (polyoxyethylene /polyoxypropylene block copolymers) and poloxamines (polyoxyethylene/polyoxypropylene block copolymers of ethylene diamine). Suitable "poloxomers" comprise block copolymers of polyoxyethylene/polyoxypropylene having the following structure:

$$HO - (CH_2 - CH_2 - O)_x - (CHCH_3 - CH_2 - O)_y - (CH_2 - CH_2 - O)_z - OH$$

wherein x has a value ranging from about 2 to about 40, y has a value ranging from about 10 to about 50, and z has a value ranging from about 2 to about 40, and preferably x and z have the same value. These copolymers are available as Pluronic® from BASF Corp., Parsippany, NJ. Suitable poloxamers and poloxamines are available as Synperonic® from ICI Chemicals, Wilmington, DE, or as Tetronic® from BASF Corp., Parsippany, NJ.

Also suitable for use herein as hydroxyl-free plasticizers are other hydrogen bond forming organic compounds which do not have hydroxyl group, including urea and urea derivatives; anhydrides of sugar alcohols such as sorbitan; animal proteins such as gelatin; vegetable proteins such as sunflower protein, soybean proteins, cotton seed proteins; and mixtures thereof. All of the plasticizers may be use alone or in mixtures thereof.

Typically, the hydroxyl plasticizer comprises from about 5 wt% to about 70 wt%, more preferably from about 15 wt% to about 60 wt%, most preferably from about 30 wt% to about 40 wt% of the starch composition. The hydroxyl-free plasticizer typically comprises from about 0.1 wt% to about 70 wt%, preferably from about 5 wt% to about 65 wt%, more preferably from about 20 wt% to about 60 wt% of the starch composition.

In one embodiment, a mixture of the hydroxyl and hydroxyl-free plasticizers is used, wherein the hydroxyl plasticizers are sugars, such as sucrose, fructose, and sorbitol, and the hydroxyl-free plasticizers are urea and urea derivatives. It is found that urea and its derivatives in the starch composition of the present invention have a strong tendency to crystallize, that is, crystallization of urea and its derivatives occurs even under fast cooling condition such as melt blowing, spun bonding, melt extrusion, wet spinning, and the like. Therefore, urea and urea derivatives may be used as solidifying agents for modifying or controlling the solidification rate of the starch composition of the present invention. In a preferred embodiment, a mixture of sucrose and urea is added to the starch/polymer composition in an amount effective to achieve the desired melt processability and cooling rate.

Diluents may be added to the starch compositions of the present invention to adjust the melt shear viscosity and enhance the melt spinnability of the starch compositions. Generally, the melt shear viscosity decreases in a nonlinear manner as the polar solvent content is increased. Typically, the polar solvent is added in an amount from about 5 wt% to about 40 wt%, preferably from about 7 wt% to about 30 wt%, more preferably from about 10 wt% to about 20 wt%, of the total composition.

Suitable for use herein as diluents are polar solvents having a solubility parameter $\delta$ ranging from about 19 to about 48 $MPa^{1/2}$, preferably from about 24 to about 48 $MPa^{1/2}$, and more preferably from about 28 to about 48 $MPa^{1/2}$. Nonlimiting examples include water, C1-C18 linear or branched alcohols, DMSO (dimethyl sulphoxide), formamide and derivatives such as N-methyl formamide, N-ethyl formamide, acetamide and derivatives such as methyl acetamide, Cellosolv® (a glycol alkyl ether) and derivatives, such as butyl Cellosolv®, benzyl Cellosolv®, Cellosolv® acetate (all Cellosolv® and derivatives are available from J. T. Baker, Phillipsburg, NJ), hydrazine, and ammonia. It is also known that the $\delta$ value of a solvent mixture can be determined by volume-averaging the $\delta$ values of the individual solvents. Therefore, mixed solvents having $\delta$ values within the above-identified range (i.e., from about 19 to about 48 $MPa^{1/2}$) are also suitable for use herein. For example, a mixed solvent of DMSO/water having a composition of 90/10 v/v would have a $\delta$ value of about 28.5; such a mixed solvent system is suitable for use herein.

It is found that polar solvents capable of forming hydrogen bonding are more effective in lowering the melt viscosity of the composition. As such, a lower amount of the polar solvent is sufficient to adjust the viscosity to the desired range for melt spinning. Using a lower amount of the polar solvent provides a further advantage of reducing the need for an evaporation step during or subsequent to the melt processing step, which results in operating cost advantages such as lower energy consumption, AND lower solvent recovery costs, as well as lower costs for environmental/regulatory compliance.

The starch composition may optionally include liquid or volatile processing aids which function mainly as viscosity modifiers of the melt compositions. The processing aid is substantially volatized and removed during the melt processing stage such that only a residual/trace amount remains in the final product. Thus, they do not adversely affect the strength, modulus or other properties of the final product. The polar solvents disclosed above may also function as volatile processing aids. Other nonliminting examples include carbonates such as sodium bicarbonate.

Optionally, other ingredients may be incorporated into the spinnable starch composition to modify the processability and/or to modify physical properties such as elasticity, tensile strength and modulus of the final product. Nonlimiting examples include cross-linking agents, emulsifiers, surfactants, lubricants, other processing aids, optical brighteners, antioxidants, flame retardants, dyes, pigments, fillers, proteins and their alkali salts, biodegradable synthetic polymers, waxes, low melting synthetic thermoplastic polymers, tackifying resins, extenders, and mixtures thereof. These optional ingredients may be present in quantities ranging from 0.1% to 70% by weight of the composition.

Exemplary biodegradable synthetic polymers include polycaprolactone; polyhydroxyalkanoates including polyhydroxybutyrates, and polyhydroxyvalerates; polylactides; and mixtures thereof.

Other additives are typically included with the starch polymer as a processing aid and to modify physical properties such as elasticity, dry tensile strength, and wet strength of the extruded fibers. Additives are typically present in quantities ranging from 0.1% to 70% by weight on a non-volatiles basis. Preferred additives are urea, urea derivatives, cross-linking agents, emulsifiers, surfactants, lubricants, proteins and their alkali salts, biodegradable synthetic polymers, waxes, low melting synthetic thermoplastic polymers, tackifying resins, extenders, and mixtures thereof. Preferred biodegradable synthetic polymers include polycaprolactone, polyhydroxybutyrates, polyhydroxyvalerates, polylactides, and mixtures thereof. Other preferred additives and associated properties include optical brighteners, antioxidants, flame retardants, dyes, pigments, and fillers. For the present invention, a preferred additive is urea in quantities ranging from 20% to 60% by weight.

Suitable extenders for use herein include gelatin, vegetable proteins such as sunflower protein, soybean proteins, cotton seed proteins, and water soluble polysaccharides; such as alginates, carrageenans, guar gum, agar, gum arabic and related gums, pectin, water soluble derivatives of cellulose, such as alkylcelluloses, hydroxyalkylcelluloses, carboxymethylcellulose, etc. Also, water soluble synthetic polymers, such as polyacrylic acids, polyacrylic acid esters, polyvinylacetates, polyvinylalcohols, polyvinylpyrrolidone, etc., may be used.

Lubricant compounds may further be added to improve the flow properties of the starch material during the processes used for producing the present invention. The lubricant compounds can include animal or vegetable fats, preferably in their hydrogenated form, especially those which are solid at room temperature. Additional lubricant materials include mono-glycerides and di-glycerides and phosphatides, especially lecithin. For the present invention, a preferred lubricant compound includes the mono-glyceride, glycerol mono-stearate.

Further additives including inorganic fillers such as the oxides of magnesium, aluminum, silicon, and titanium may be added as inexpensive fillers or processing aides. Additionally, inorganic salts, including alkali metal salts, alkaline earth metal salts, phosphate salts, etc., may be used as processing aides.

Other additives may be desirable depending upon the particular end use of the product contemplated. For example, in products such as toilet tissue, disposable towels, facial tissues and other similar products, wet strength is a desirable attribute. Thus, it is often desirable to add to the starch polymer cross-linking agents known in the art as "wet strength" resins.

A general dissertation on the types of wet strength resins utilized in the paper art can be found in TAPPI monograph series No. 29, Wet Strength in Paper and Paperboard, Technical Association of the Pulp and Paper Industry (New York, 1965). The most useful wet strength resins have generally been cationic in character. Polyamide-epichlorohydrin resins are cationic polyamide amine-epichlorohydrin wet strength resins which have been found to be of particular utility. Suitable types of such resins are described in U.S. Patent Nos. 3,700,623, issued on October 24, 1972, and 3,772,076, issued on November 13, 1973, both issued to Keim. One commercial source of a useful polyamide-epichlorohydrin resin is Hercules, Inc. of Wilmington, Delaware, which markets such resins under the mark Kymene®.

Glyoxylated polyacrylamide resins have also been found to be of utility as wet strength resins. These resins are described in U.S. Patent Nos. 3,556,932, issued on January 19, 1971, to Coscia, et al. and 3,556,933, issued on January 19, 1971, to Williams et al. One commercial source of glyoxylated polyacrylamide resins is Cytec Co. of Stanford, CT, which markets one such resin under the mark Parez® 631 NC.

It is found that when suitable cross-linking agent such as Parez® is added to the starch composition of the present invention under acidic condition, The composition is rendered water insoluble. That is, the water solubility of the composition, as tested by the Test Method described hereinafter, is less than 30%, preferably less than 20%, more preferably less than 10% and most preferably less than 5%. The products such as fibers and films made from such a composition are also water insoluble.

Still other water-soluble cationic resins finding utility in this invention are urea formaldehyde and melamine formaldehyde resins. The more common functional groups of these polyfunctional resins are nitrogen containing groups such as amino groups and methylol groups attached to nitrogen. Polyethylenimine type resins may also find utility in the present invention. In addition, temporary wet strength resins such as Caldas® 10 (manufactured by Japan Carlit) and CoBond® 1000 (manufactured by National Starch and Chemical Company) may be used in the present invention.

For the present invention, a suitable cross-linking agent is added to the composition in quantities ranging from about 0.1% by weight to about 10% by weight, more preferably from about 0.1% by weight to about 3% by weight.

The Rheology of The Starch Compositions

The rheological behavior of the starch composition is an important consideration for selecting suitable materials and fabrication equipment/processes. Many factors contribute to the rheological behavior of the starch composition, including the amount and the type of polymeric components used, the molecular weight and molecular weight distribution of the components, the amount and type of additives (e.g., plasticizers, processing aids), the processing conditions such as temperature, pressure, rate of deformation, and relative humidity, and in the case of non-Newtonian materials, the deformation history (i.e., a time or strain history dependence).

The starch composition of the present invention typically has a high solid content (i.e., a concentration above a critical concentration C*) such that a dynamic or fluctuating entangled network is formed wherein the starch molecules and the high polymers become associated and disassociated temporally. The association may be in the form of physical entanglements, van der Waals forces, or chemical interactions such as hydrogen bonding. The starch composition having the entangled network structure exhibits melt flow behavior typical of a non-Newtonian fluid.

The starch composition of the present invention may exhibit a strain hardening behavior, that is, the extensional viscosity increases as the strain or deformation increases. Typically, a Newtonian fluid exhibit a linear relationship between stress/force and strain. That is, there is no strain hardening behavior in a Newtonian fluid. On the other hand, a non-Newtonian fluid may exhibiting an increase in force at higher strain (i.e, strain hardening) while still exhibit a linear force - strain relationship at lower strain (i.e, Newtonian-like).

The strain experienced by a fluid element in a non-Newtonian fluid is dependent on its kinematic history, that is

$$\varepsilon = \int_0 \varepsilon^\bullet(t') \, \partial t'$$

This time or history dependent strain is called the Hencky strain ($\varepsilon_H$). For an ideal homogeneous uniaxial elongation, the strain rate experienced by every fluid element is equal to the strain imposed by the applied stress, such as the stresses applied externally by the instrument, device or process. In such an ideal case, the Hencky strain correlates directly with the sample deformation/elongation

$$\varepsilon_H = ln \, (L/L_o)$$

Such an ideal strain response to applied stress is most often observed in Newtonian fluids.

The Trouton ratio (Tr) is often used to express the extensional flow behavior. The Trouton ratio is defined as the ratio between the extensional viscosity ($\eta_e$) and the shear viscosity ($\eta_s$),

$$Tr = \eta_e(\dot{\varepsilon},t) / \eta_s$$

wherein the extensional viscosity $\eta_e$ is dependent on the deformation rate ($\varepsilon^\bullet$) and time (t). For a Newtonian fluid, the uniaxial extension Trouton ratio has a constant value of 3. For a non-Newtonian fluid, the extensional viscosity is dependent on the deformation rate ($\varepsilon^\bullet$) and time (t).

Shear viscosity ($\eta_s$) relates to the melt processability of the starch composition using standard polymer processing techniques, such as extrusion, blow molding, compression molding, injection molding and the like. A starch composition having a shear viscosity, measured according to the Test Method disclosed hereinafter, of less than about 30 Pa•s, preferably from about 0.1 to about 10 Pa•s, more preferably from about 1 to about 8 Pa•s, is useful in the melt attenuation processes herein. Some starch compositions herein may have low melt viscosity such that they may be mixed, conveyed, or otherwise processed in traditional polymer processing equipment typically used for viscous fluids, such as a stationary mixer equipped with metering pump and spinneret. The shear viscosity of the starch composition may be effectively modified by the molecular weight and molecular weight distribution of the starch, the molecular weight of the high polymer, and the amount of plasticizers and/or solvents used. It is found that reducing the average molecular weight of the starch is an effective way to lower the shear viscosity of the composition.

It is generally known that melt shear viscosity is a material property useful for evaluating melt processability of the material in traditional thermoplastic processes such as injection molding or extrusion. For conventional fiber spinning thermoplastics such as polyolefins, polyamides and polyesters, there is a strong correlation between shear viscosity and extensional viscosity of these conventional thermoplastic materials and blends thereof. That is, the spinnability of the material can be determined simply by the melt shear viscosity, even though the spinnablity is a property controlled primarily by melt extensional viscosity. The correlation is quite robust such that the fiber industry has relied on the melt shear viscosity in selecting and formulating melt spinnable materials. The melt extensional viscosity has rarely been used as an industrial screening tool.

It is therefore surprising to find that the starch compositions of the present invention do not exhibit such a correlation between shear and extensional viscosities. Specifically, when a high polymer selected according to the present invention is added to a starch composition, the shear viscosity of the composition remains relatively unchanged, or even decreases slightly. Based on conventional wisdom, such a starch composition would exhibit decreased melt processability and would not be suitable for melt extensional processes. However, it is surprisingly found that the starch composition herein shows a significant increase in extensional viscosity when even a small amount of high polymer is added. Consequently, the starch composition herein is found to have enhanced melt extensibility and is suitable for melt extensional processes (e.g., blow molding, spun bonding, blown film molding, foam molding, and the like).

Extensional or elongational viscosity ($\eta_e$) relates to melt extensibility of the composition, and is particularly important for extensional processes such as fiber, film or foam making. The extensional viscosity includes three types of deformation: uniaxial or simple extensional viscosity, biaxial extensional viscosity, and pure shear extensional viscosity. The uniaxial extensional viscosity is important for uniaxial extensional processes such as fiber spinning, melt blowing, and spun bonding. The other two extensional viscosities are important for the biaxial extension or forming processes for making films, foams, sheets or parts. It is found that the properties of the high polymers have a significant effect on melt extensional viscosity. The high polymers useful for enhancing the melt extensibility of the starch composition of the present invention are typically high molecular weight, substantially linear polymers. Moreover, high polymers that are substantially compatible with starch are most effective in enhancing the melt extensibility of the starch composition.

It has been found that starch compositions useful for melt extensional processes typically have their extensional viscosity increased by a factor of at least 10 when a selected high polymer is added to the composition. Typically, the starch compositions of present invention show an increase in the extensional viscosity of about 10 to about 500, preferably of about 20 to about 300, more preferably from about 30 to about 100, when a selected high polymer is added.

It has also been found that melt processable compositions of the present invention typically have a Trouton ratio of at least about 3. Typically, the Trouton ratio ranges from about 10 to about 5,000, preferably from about 20 to about 1,000, more preferably from about 30 to about 500, when measured at 90 °C and 700 s$^{-1}$.

When the starch composition of the present composition is subjected to an uniaxial extensional process, a draw ratio, expressed in ($D_o^2/D^2$) wherein $D_o$ is the diameter of filament before drawing and D is the diameter of the drawn fiber, greater than 1000 can be easily achieved. The starch composition of the present invention typically achieves a draw ratio from about 100 to about 10,000, preferably greater than about 1,000, more preferably greater than about 3,000 and most preferably greater than about 5,000. More specifically, the starch composition of the present invention has sufficient melt extensibility to be melt drawn to fine fibers having a finite average diameter of less than 50 microns, preferably less than 25 microns, more preferably less than 15 microns, even more preferably less than 10 microns, and most preferably less than 5 microns.

When the starch composition of the present invention is subjected to a biaxial extensional process, the enhanced melt extensibility of the composition allows it to be melt drawn to films having a finite average caliper of less than 0.8 mils, preferably less than 0.6 mils, more preferably less than 0.4 mils, even more preferably less than 0.2 mils, and most preferably less than 0.1 mils.

The starch composition herein is processed in a flowable state, which typically occurs at a temperature at least equal to or higher than its melting temperature. Therefore, the processing temperature range is controlled by the melting temperature of the starch composition, which is measured according to the Test Method described in detail herein. The melting temperature of the starch composition herein ranges from about 80 to 180°C, preferably from about 85 to about 160°C, and more preferably from about 90 to about 140°C. It is to be understood that some starch compositions may not exhibit pure "melting" behavior. As used herein, the term "melting temperature" means the temperature or the range of temperature at or above which the composition melts or softens.

Exemplary, uniaxial extensional processes suitable for the starch compositions include melt spinning, melt blowing, and spun bonding. These processes are described in detail in U. S. Patent No. 4,064,605, issued on December 27, 1977 to Akiyama et al.; U.S. Patent No. 4,418,026, issued on November 29, 1983 to Blackie et al.; U. S Patent No. 4,855,179, issued on August 8, 1989 to Bourland et al.; U. S. Patent No. 4,909,976, issued on March 20, 1990 to Cuculo et al.; U. S. Patent No. 5,145,631, issued on September 8, 1992 to Jezic; U.S. Patent No. 5,516,815, issued on May 14, 1996 to Buehler et al.; and U.S. Patent No. 5,342,335, issued on August 30,1994 to Rhim et al. The resultant products may find use in filters for air, oil and water; vacuum cleaner filters; furnace filters; face masks; coffee filters, tea or coffee bags; thermal insulation materials and sound insulation materials; nonwovens for one-time use sanitary products such as diapers, feminine pads, and incontinence articles; biodegradable textile fabrics for improved moisture absorption and softness of wear such as microfiber or breathable fabrics; an electrostatically charged, structured web for collecting and removing dust; reinforcements and webs for hard grades of paper, such as wrapping paper, writing paper, newsprint, corrugated paper board, and webs for tissue grades of paper such as toilet paper, paper towel, napkins and facial tissue; medical uses such as surgical drapes, wound dressing, bandages, dermal patches and self-dissolving sutures; and dental uses such as dental floss and toothbrush bristles. The fibrous web may also include odor absorbants, termite repellants, insecticides, rodenticides, and the like, for specific uses. The resultant product absorbs water and oil and may find use in oil or water spill clean-up, or controlled water retention and release for agricultural or horticultural applications. The resultant starch fibers or fiber webs may also be incorporated into other materials such as saw dust, wood pulp, plastics, and concrete, to form composite materials, which can be used as building materials such as walls, support beams, pressed boards, dry walls and backings, and ceiling tiles; other medical uses such as casts, splints, and tongue depressors; and in fireplace logs for decorative and/or burning purpose.

The melt rheological behavior of the present starch composition also makes it suitable for use in conventional thermoplastic processes that involves biaxial extension of the material. By having the proper melt shear viscosity and biaxial extensional viscosity, the starch compositions of the present invention may substantially reduce the occurrence of tearing, surface defects, and other breakdowns or defects that interrupt continuous processes and produce unsatisfactory products. These processes include blow molding, blown film extrusion or coextrusion, vacuum forming, pressure forming, compression molding, transfer molding and injection molding. Nonlimiting examples of these processes are described in details in U.S. Patent No. 5,405,564, issued on April 11, 1995 to Stepto et al.; U.S. Patent No. 5,468,444, issued on November 21, 1995 to Yazaki et al.; U.S. Patent No. 5,462,982, issued on October 31, 1995 to Bastioli et al. The articles produced by these processes include sheets, films, coatings, laminates, pipes, rods, bags, and shaped articles (such as bottles, containers). The articles may find use as bags such as shopping bags, grocery bags, and garbage bags; pouches for food storage or cooking; microwavable containers for frozen food; and pharmaceutical uses such as capsules or coatings for medicine. The films may be substantially transparent for use as food wraps, shrink wraps or windowed envelopes. The films may also be further processed for use as an inexpensive, biodegradable carrier for other materials such as seeds or fertilizers. Adhesives may be applied to the films or sheets for other uses such as labels.

The starch compositions of the present invention may also be made into a foamed structure by controlled removal of the volatile components (e.g., water, polar solvents). However, foaming or expanding agents are generally incorporated to produce articles having foamed or porous internal structure. Exemplary foaming or expanding agents include carbon dioxide, n-pentane, and carbonate salts such as sodium bicarbonate, either alone or in combination with a polymeric acid which has lateral carboxyl groups (e.g., polyacrylic acid, ethylene-acrylic copolymer). Nonlimiting examples of the foaming and forming processes are described in U. S. Patent No. 5,288,765, issued on February 22, 1994 to Bastioli et al.; U.S. Patent No. 5,496,895, issued on March 5, 1996 to Chinnaswamy et al.; U.S. Patent No. 5,705,536, issued on January 6, 1998 to Tomka; and U.S. Patent No. 5,736,586, issued on April 7, 1998 to Bastioli et al. The resultant products may find use in egg cartons; foamed cups for hot beverages; containers for fast food; meat trays; plates and bowls for one-time use such as at picnic or parties; packaging materials, either loose-fill or molded to conform to the packed article (e.g., a computer shipping package); thermal insulation materials; and noise insulation or sound proofing materials.

(2) Exemplary Processes Extruder Apparatus

The apparatus for carrying out the process of the invention consists of an extruder having

a. first inlet chamber containing at least one conveying element,
b. a heated receiving chamber downstream of said first chamber and containing at least one conveying element;
c. a heated destructurization chamber, downstream of said second chamber, containing kneading and retaining elements;
d. a heated degassing chamber under reduced pressure downstream of said destructurization chamber and said degassing chamber containing at least one conveying element, and
e. a heated extrusion chamber downstream of said degassing chamber being under elevated pressure and having at least one conveying element.

Furthermore, the extruder preferably has at least one delivery device for solids for process step a, a liquid metering device for process step b, a degassing fitting for process step d, and a die for process step e. A twin screw extruder having closely meshing screws which run in the same direction is preferred.

For the present invention, the starch material can have a total water content, i.e. water of hydration plus added water, in the range of about 5 to about 40%; preferably in the range of about 10 to about 20%. The starch material is heated to elevated temperatures sufficient to form a pseudo-thermoplastic melt. Such temperature is typically higher than the glass transition and/or melting temperature of the formed material. For the present invention, the glass transition temperatures are at least about minus -30°C, preferably in the range of about -30°C to about 150°C, more preferably in the range of about -30°C to about 100°C, and most preferably in the range of about -30°C to about 25°C. The melting temperature is preferably in the range of about 100°C to about 180°C. The pseudo-thermoplastic melts of the invention are polymeric fluids having a shear rate dependent viscosity, as known in the art. The viscosity decreases with increasing shear rate as well as with increasing temperature.

The starch material is heated preferably in a closed volume in the presence of a low concentration of water to convert the starch material to a pseudo-thermoplastic melt. A closed volume can be a closed vessel or the volume created by the sealing action of the feed material as happens in the screw of extrusion equipment. Pressures created in a closed vessel will include pressures due to the vapor pressure of water as well as pressures generated due to compression of materials in the screw-barrel of the extruder.

A chain scission catalyst, which reduces the molecular weight by splitting the glycosidic bonds in the starch macromolecules resulting in a reduction of the average molecular weight of the starch, may be used to reduce the viscosity of the pseudo-thermoplastic melt. Suitable catalysts include inorganic and organic acids. Suitable inorganic acids include hydrochloric acid, sulfuric acid, nitric acid, phosphoric acid, and boric acid as well as the partial salts of polybasic acids, e.g. $NaHSO_4$ or $NaH_2PO_4$ etc. Suitable organic acids include formic acid, acetic acid, propionic acid, butyric acid, lactic acid, glycolic acid, oxalic acid, citric acid, tartaric acid, itaconic acid, succinic acid, and other organic acids known in the art, including partial salts of the polybasic acids. For the present invention, the preferred catalysts are hydrochloric acid, sulfuric acid, and citric acid, including mixtures thereof.

The reduction of the molecular weight of the non-modified starch used is by a factor of 2 to 5000, preferably by a factor of 4 to 4000. The concentration of catalysts is in the range of $10^{-6}$ to $10^{-2}$ mole of catalyst per mole of anhydro-glucose unit, preferably between $0.1 \times 10^{-3}$ to $5 \times 10^{-3}$ mole of catalyst per mole of anhydro-glucose unit of starch.

The following examples illustrate the type of extrusion equipment and operating parameters for producing starch fibers.

**Example 1**

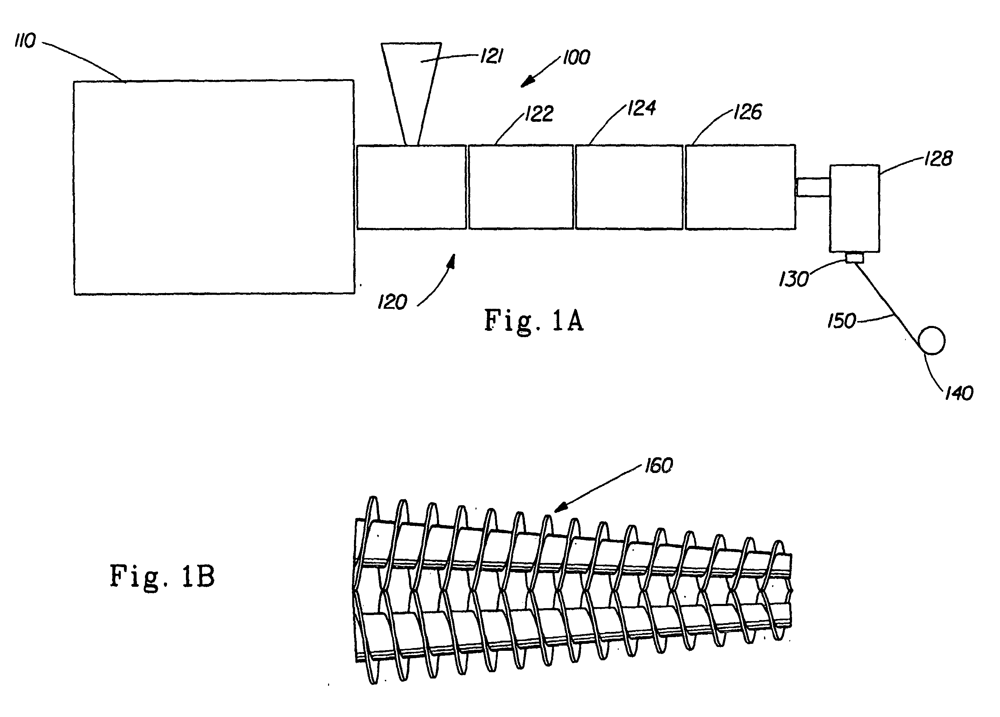

The purpose of this example is to illustrate starch fibers extruded at a particular cross section and subsequently drawn to a reduced cross section. Drawn pseudo-thermoplastic starch fibers were produced using a torque rheometer assembly 100 illustrated in Figure 1a. The torque rheometer assembly 100 includes a drive unit 110 (manufactured by Haake GmbH, model Rheocord 90), a barrel 120 partitioned into four temperature zones 122,124,126 and 128, a feed port 121, a single capillary die 130, and a simple mandrel winder 140. Twin screw elements 160 (model TW100, from Haake GmbH), depicted in Figure 1b, are attached to the drive unit 110 and disposed within the barrel 120. A capillary die was made to fit the die 130, with an orifice diameter of 0.5 mm and a length of 5.6 mm. The mandrel rewinder 140 comprises a 3 inch core mounted to a simple DC driven 3 inch diameter shaft. The 3 inch core can achieve surface speeds from 150 to 2000 fpm.

Raw materials utilized included the following:

45% by weight Durabond A Corn Starch from National Starch
25% by weight Water
15% by weight Urea available from Aldrich Chemicals
15% by weight Sorbitol available from Aldrich Chemicals

All raw materials were mixed off-line until a slurry was formed. The slurry was then manually fed into the feed port 121 of the torque rheometer assembly 100.

The settings on the torque rheometer were as follows:

| | |
|---|---|
| RPM | 50 |
| Barrel Temperature | 110 °C |
| Die Temperature | 105 °C |
| Feed Rate | 1.7 grams/minute |

After running the rheometer for approximately 20 minutes, the process stabilized and a single pseudo-thermoplastic starch fiber 150 exited the die 130. The single fiber 150 was manually wound around the mandrel winder 140. The winder 140 was then slowly sped up to 900 feet/minute surface speed in order to draw the fiber 150 increasing the fiber length and decreasing the cross sectional area. The diameter of the wound fiber 150 was between 70 and 90 microns.

**Example 2**

The purpose of this example is to illustrate how starch fibers can be arranged to form a starch fiber structure. The pseudo-thermoplastic starch fibers of Example 1 were cut into 8 mm length staple fibers. The starch staple fibers at a basis weight of 55 g/m² were air laid onto a papermaking forming fabric as described in U.S. Patent No. 4,637,859, with the fabrics of U.S. Patent Nos. 5,857,498, 5,672,248, 5,211,815 and 5,098,519, also being judged suitable for this purpose.. The fibers were misted with water at a level of 20%, based on the weight of the fibers, and then dried at an elevated temperature to produce a bonded starch fiber structure.

**Example 3 :Starch Fibers Extruded Using A Vented Twin Screw Extruder**

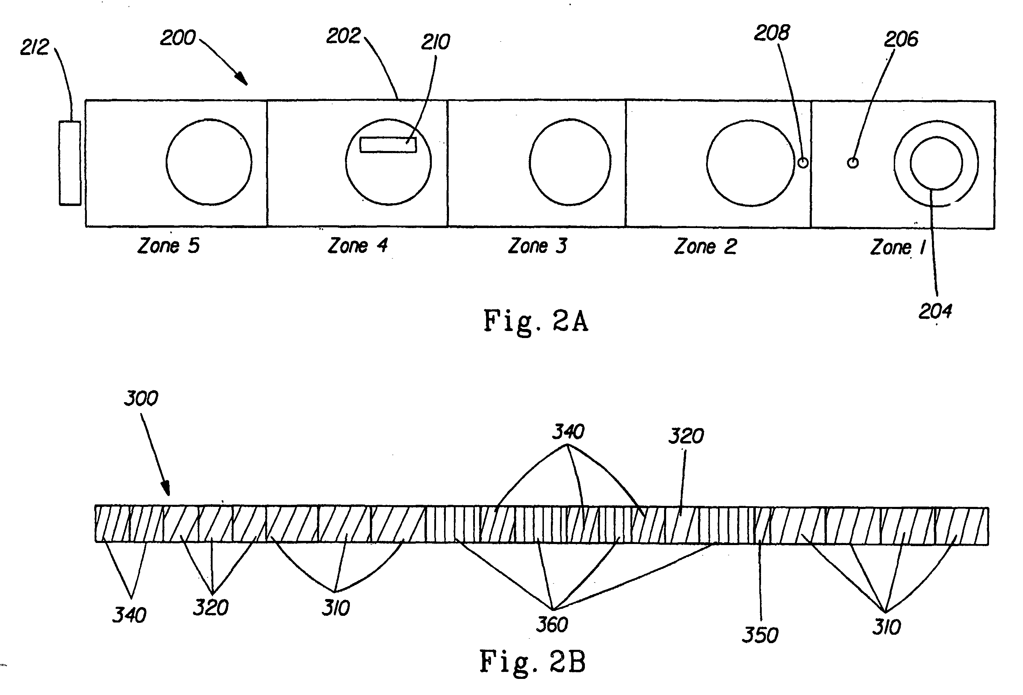

The purpose of this example is to illustrate a vented twin screw extruder configuration, depicted in Figure 2a, used to make starch fibers for the present invention. Starch fibers are made using a an APV Baker (Peterborough, England) twin screw extruder 200, a capillary die 212, and a winder (not shown).

As show in Figure 2a, the twin screw extruder comprises a barrel 202 that is separated into five zones. The barrel 202 encloses the extrusion screws and mixing elements and serves as a containment vessel during the extrusion process. A solid feed port 204 is disposed in zone 1 and liquid feed ports 206 and 208 are disposed in zone 1 and zone 2. A vent 210 is included in zone 4 for venting the pseudo-thermoplastic melt to decrease the water content of the mixture prior to extrusion through the die 212.

The screw and mixing element configuration 300 for the twin screw extruder 200 is illustrated in Figure 2b. The twin screw extruder comprises a plurality of twin lead screws (TLS) and single lead screws (SLS) installed in series. Screw elements are characterized by the number of continuous leads and the pitch of these leads.

A lead is a flight (at a given helix angle) which wraps the core of the screw element. The number of leads indicates the number of flights wrapping the core at any given location along the length of the screw. Increasing the number of leads reduces the volumetric capacity of the screw and increase the pressure generating capability of the screw.

The pitch of the screw is the distance needed for a flight to complete one revolution of the core. It is expressed as the number of screw element diameters per one complete revolution of a flight. Decreasing the pitch of the screw increase the pressure generated by the screw and decreases the volumetric capacity of the screw.

The length of a screw element is reported as the ratio of length of the element divided by the diameter of the element.

This example uses TLS and SLS. Screw element 310 is a TLS with a 1.0 pitch and a 1.5 length ratio. Screw element 320 is a TLS with a 1.0 pitch and a 1.0 L/D ratio. Screw element 340 is a SLS with a ¼ pitch and a 1.0 length ratio. Screw element 350 is a SLS and a ¼ pitch and a ½ length ratio.

Bilobal paddles 360 serving as mixing elements are also included in a series with the SLFS and TLFS screws in order to enhance mixing. Various configurations of bilobal paddles 360 and reversing elements 340 and 350 are used in order to control flow and corresponding mixing time.

In zone 1, Durabond A starch and sorbitol are fed into the solid feed port 204 and urea is fed into the liquid port 208 forming a mixture with a 60/20/20 weight ratio. These materials are combined inside the extruder with water added at the liquid feed port 206 to form a pseudo-thermoplastic melt. The temperature, pressure, and corresponding function of each zone are provided in Table I.

Table I

| Zone | Temperature (deg F) | Pressure (gauge PSI) | Description of Screw |
|---|---|---|---|
| 1 | 70 | 0 | Feeding |
| 2 | 193 | 34 | Mixing |
| 3 | 268 | 0 | Mixing |
| 4 | 210 | 0 | Pressure Decreasing Conveying |
| 5 | 205 | 0-10 | Pressure Generating |
| Die | 194 | 430 | Shaping |

**Example 4 :Starch Fibers Extruded With A Non-Vented Twin Screw Extruder**

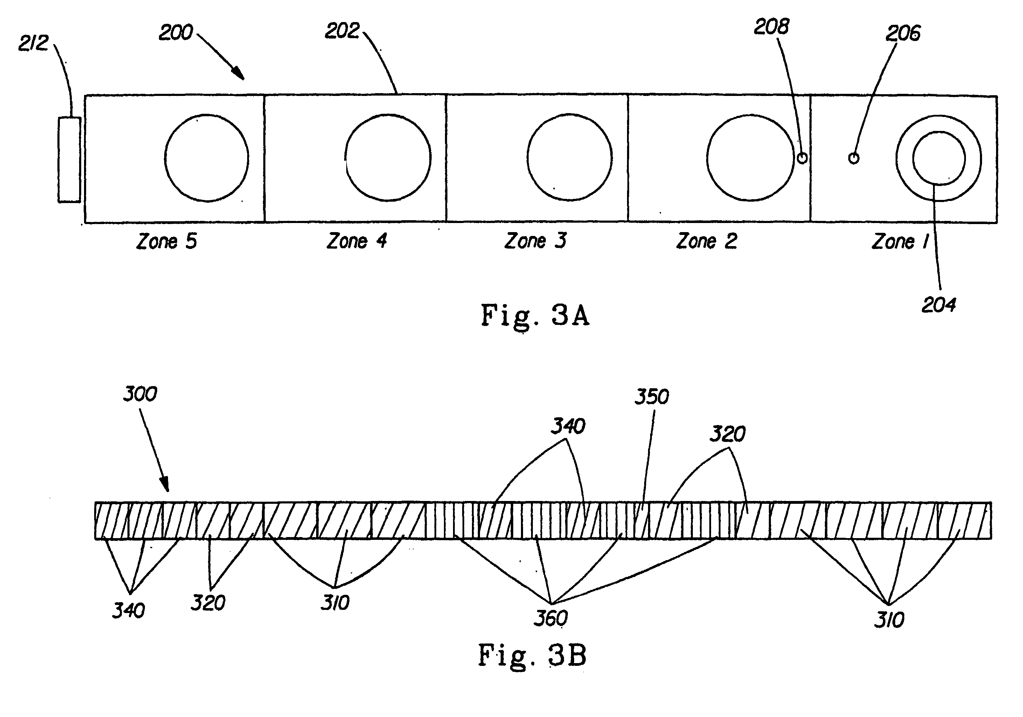

The purpose of this example is to illustrate a non-vented twin screw extruder configuration, depicted in Figure 3a, used to make starch fibers for the present invention. Starch fibers are made using an APV Baker (Peterborough, England) twin screw extruder 200, a capillary die 212, and a winder (not shown).

The non-vented twin screw extruder configuration is illustrated in Figure 3a. The twin screw extruder comprises a barrel 202 that is separated into five zones. The barrel 202 encloses the extrusion screws and mixing elements and serves as a containment vessel during the extrusion process. A solid feed port 204 is disposed in zone 1 and liquid feed ports 206, and 208 are disposed in zone 1 and zone 2.

The screw and mixing element configuration for the twin screw extruder is illustrated in Figure 3b.

In zone 1, Durabond A starch and sorbitol are fed into the solid feed port 204 and urea is fed into the liquid port 208 forming a mixture with a 60/20/20 weight ratio. These materials are combined inside the extruder with water added at the liquid feed port 206 to form a pseudo-thermoplastic melt. The temperature, pressure, and corresponding function of each zone are provided in Table II.

Table II

| Zone | Temperature (deg F) | Pressure (gauge PSI) | Description of Screw |
|------|--------------------|--------------------|---------------------|
| 1 | 70 | 0 | Feeding |
| 2 | 180 | 0 | Mixing |
| 3 | 260 | 0 | Mixing |
| 4 | 215 | 0 | Pressure Decreasing Conveying |
| 5 | 193 | 30 | Pressure Generating |
| Die | 172 | 150 | Shaping |

**Example 5 : Foamed Starch Fibers Extruded With A Non-Vented Twin Screw Extruder**

The purpose of this example is to illustrate the various zones of a twin screw extruder without a vent and the operating parameters associated with each zone for producing foamed starch fibers which are lower in density and having a higher absorbent capacity relative to non-foamed starch fibers. Foamed starch fibers are made using a fiber making apparatus comprising the twin screw extruder configuration depicted in Figures 3a and 3b.

In zone 1, Durabond A starch and sorbitol are fed into the solid feed port 204 and urea is fed into the liquid port 208 forming a mixture with a 60/20/20 weight ratio. These materials are combined inside the extruder with water added at the liquid feed port 206 to form a pseudo-thermoplastic melt. The temperature, pressure, and corresponding function of each zone are provided in Table III.

Table III

| Zone | Temperature (deg F) | Pressure (gauge PSI) | Description of Screw |
|------|--------------------|--------------------|---------------------|
| 1 | 70 | 0 | Feeding |
| 2 | 180 | 0 | Mixing |
| 3 | 260 | 0 | Mixing |
| 4 | 240 | 0 | Pressure Decreasing Conveying |
| 5 | 220 | 30 | Pressure Generating |
| Die | 225 | 150 | Shaping |

Pseudo-thermoplastic Starch Melt Fiber Spinning

The production of fibers according to the invention from the pseudo-thermoplastic melt compositions occurs by the usual melt spinning processes. Devices for producing non-woven thermoplastic fabric structures from extruded polymers are well known in the art. Extruded polymers under pressure, are forced through a spinneret forming a vertically oriented curtain of downward advancing fibers. The fibers are quenched with air in conjunction with a suction-type drawing or attenuating air slot. U.S. Pat. No. 5,292,239 issued to Zeldin, et al., March 8, 1994, discloses a device that reduces significant turbulence in the air flow in order to uniformly and consistently apply a drawing force to the fibers.

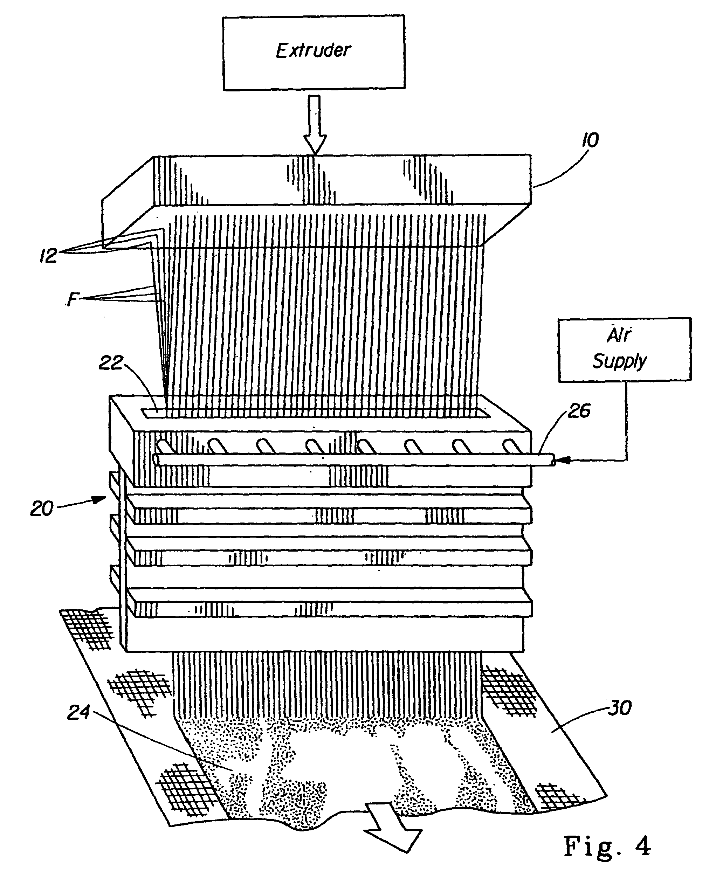

For the present invention, structures are produced from a mixture comprising starch, water, plasticizers, and other optional additives. As shown in Figure 4, the mixture is converted to a pseudo-thermoplastic melt in an extruder and conveyed through a spinneret 10 to a drawing unit 20 forming a vertically oriented curtain of downward advancing fibers F.

The spinneret 10 comprises an assembly which is known in the art. The spinneret 10 includes a plurality of nozzle bores 12 with hole diameters customary for fiber production. The spinneret assembly 10 can be adapted to the fluidity of the melt so that every nozzle bore 12 has the same rate of flow.

The drawing unit 20 comprises an open upper end 22, an open lower end 24, and an air supply manifold 26 supplying compressed air to internal nozzles (not shown) oriented in a downward direction. As compressed air flows through the internal nozzles, air is drawn into the open upper end 22 of the drawing unit 20 forming a rapidly moving stream of air flowing in the downward direction. The air stream produces a drawing force on the fibers causing them to be attenuated or stretched before exiting the open lower end 24 of the drawing unit 20.

For the present invention, the fibers exiting the drawing unit 20 can have a size ranging from 0.01 decitex to 5 decitex. Preferably, the fibers exiting have a size ranging from 0.02 decitex to 5 decitex.

Upon exiting the drawing unit 20, the fibers are deposited on a moving conveyor belt 30 to form flexible, low density structure comprising fibers. The fibers are then joined to each other through conventional techniques. A preferred process for producing structures of the present invention is described in U.S. Pat. No. 5,688,468 issued to Lu, November 18, 1997.

In addition to spunbonded structures, mono-fibers, multi-fibers, staple fibers, hollow fibers, shaped fibers, such as multi-lobal fibers and multi-component fibers can all be produced by using the compositions and methods of the present invention. The process for the production of these fibers may be in one stage with a compounding extruder producing a pseudo-thermoplastic starch melt and conveying it without cooling through a melt filter to a spinneret. Staple starch fibers may also be converted to flexible, low density structures by carding, air laying, and similar processes known in the art.

(3) Material Properties

Products such as disposable towels, toilet tissue, facial tissue, napkins and wet wipes manifest various physical characteristics which include basis weight and apparent density, both of which have been previously defined. For the present invention, the structure comprising pseudo-thermoplastic starch fibers can have a basis weight ranging from about 10 g/m$^2$ to about 450 g/m$^2$. More preferably, the structure can have a basis weight ranging from about 12 g/m$^2$ to about 150 g/m$^2$. Moreover, the structure of the present invention can have an apparent density ranging from about 0.02 g/cm$^3$ to about 0.20 g/cm$^3$; more preferably, an apparent density ranging from about 0.04 g/cm$^3$ to about 0.15 g/cm$^3$ and most preferably, an apparent density ranging from about 0.04 g/cm$^3$ to about 0.12 g/cm$^3$.

The products listed above also exhibit certain mechanical properties, particularly, strength, flexibility, and absorbency. Measures of strength include geometric mean dry tensile strength (GMDT), and geometric mean wet tensile strength (GMWT) wherein GMWT includes initial wet tensile strength and decayed wet tensile strength. Flexibility is related to stiffness and can attribute to softness. Absorbency relates to the products' ability to take up fluids as well as the capacity to retain them.

Geometric mean dry tensile strength (GMDT), previously defined, provides a measure of the dry tensile strength of the structure. The method used to determine this measure is described below. For the present invention, the structure comprising pseudo-thermoplastic starch fibers can have a GMDT ranging from about 10 g/cm to about 1200 g/cm. More preferably, the structure can have a GMDT ranging from about 30 g/cm to about 600 g/cm. Most preferably, the structure can have a GMDT ranging from about 40 g/cm to about 475 g/cm.

Geometric mean wet tensile strength (GMWT), previously defined, provides a measure of the wet tensile strength of the structure. The initial geometric mean tensile strength is the wet tensile strength of a structure after it has been immersed in water for five seconds. The method used to determine this measure is described below. For the present invention, the structure comprising pseudo-thermoplastic starch fibers can have a GMWT ranging from about 2 g/cm to about 400 g/cm. More preferably, the structure can have a GMWT ranging from about 2 g/cm to about 200 g/cm.

Geometric mean decayed wet tensile strength (GMDWT) is a measure of the wet tensile strength of the structure after being immersed in water for thirty minutes. For the present invention, the structure comprising pseudo-thermoplastic starch fibers can have a GMDWT ranging from about 0 g/cm to about 20 g/cm. More preferably, the structure can have a GMDWT ranging from about 0 g/cm to about 10 g/cm.

Softness has been described as a physiologically perceived attribute which is generally measured by expert or non-expert panel evaluations. Perceived softness can be broken down into two components; bulk softness and surface softness. Bulk softness has been correlated to sheet stiffness and flexibility while surface softness has been related to surface texture and smoothness. High softness requires flexibility. The method used for determining the total flexibility of a structure is defined below. For the present invention, the structure has a total flexibility ranging from about 1.0 g/cm to about 75 g/cm; preferably from about 2.0 g/cm to about 50 g/cm; and more preferably from about 2.0 g/cm to about 35 g/cm.

Products such as disposable towels, toilet tissue, facial tissue, napkins, and wet wipes require a certain level of absorbency. Herein, absorbency means absorbent capacity which is a measure of the amount of distilled water absorbed and retained by the structure. The method used for determining the absorbency of a structure is defined below. For the present invention, the structure has an absorbency ranging from about $1\,{}^{g_{Water}}\!/_{g_{Dry\,Stream}}$ to about $15\,{}^{g_{Water}}\!/_{g_{Dry\,Stream}}$;

preferably from about 2 $^{g\,Wax}/_{g\,Dry\,Stream}$ to about 14 $^{g\,Wax}/_{g\,Dry\,Stream}$ ; more preferably from about 3 $^{g\,Wax}/_{g\,Dry\,Stream}$ to about 13 $^{g\,Wax}/_{g\,Dry\,Stream}$ .

(4) Analytical Methods

(a) Sample Conditioning And Preparation:

Prior to testing, samples are conditioned at a relative humidity of 48% to 50% and within a temperature range of 22°C to 24°C until a moisture content of from about 5% to about 16% by weight as measured by TGA (Thermo Gravimetric Analysis) is achieved. For Thermo Gravimetric Analysis, a Hi-res. TGA2950 Termogravimetric analyzer from TA Instruments is used. Approximately 20 mg of sample is weighed into a TGA pan. Following the manufacturer's instructions, the sample and pan are inserted into the unit and the temperature is increased at a rate of 10°C/minute to 250°C. The % moisture in the sample is determined using the weight lost and the initial weight as follows:

$$\% \text{ Moisture} = \frac{\text{Start Weight - Weight @ 250}°C}{\text{Start Weight}} * 100\%$$

where all weights are in milligrams.

(b) Basis Weight

One stack of 8 plies is made from the preconditioned samples. The stack of 8 plies is cut into a 4 inch by 4 inch square. A rule die from Acme Steel Rule Die Corp. (5 Stevens St. Waterbury Conn., 06714) is used to accomplish this cutting.
For the actual measurement of the weight of the sample, a top loading balance with a minimum of 0.01 g readability is used. The stack of 8 plies is laid on the pan of the top loading balance. The balance is protected from air drafts and other disturbances using a draft shield. Weights are recorded when the readings on the balance become constant. Weights are measured in grams.
The weight reading is divided by the number of plies tested. The weight reading is also divided the weight reading by the area of the sample which is normally 16 square inches, which is approximately equal to 0.0103 square meters.
The unit of measure for basis weight as used herein is grams/square meter. This is calculated using the 0.0103 square meter area noted above.

(c) Caliper

Preconditioned samples are cut to a size greater than the size of the foot used to measure the caliper. The foot to be used is a circle with an area of 3.14 square inches.
The sample is placed on a horizontal flat surface and confined between the flat surface and a load foot having a horizontal loading surface, where the load foot loading surface has a circular surface area of about 3.14 square inches and applies a confining pressure of about 15 g/square cm (0.21 psi) to the sample. The caliper is the resulting gap between the flat surface and the load foot loading surface. Such measurements can be obtained on a VIR Electronic Thickness Tester Model II available from Thwing-Albert, Philadelphia, Pa. The caliper measurement is repeated and recorded at least five times. The result is reported in millimeters.
The sum of the readings recorded from the caliper tests is divided by the number of readings recorded. The result is reported in millimeters (mm).

(d) Dry Tensile Strength

The dry tensile strength is determined on one inch wide strips of sample using a Thwing-Albert Intelect II Standard Tensile Tester (Thwing-Albert Instrument Co.; 10960 Dutton Rd., Philadelphia, Pa., 19154). This method is intended for use on finished paper products, reel samples, and unconverted stocks.
Two stacks of 8 plies are made from the preconditioned samples. From one of these stacks of 8 plies, four strips are cut 1 inch by 7 inch with the long 7 inch dimension running parallel to the machine direction. Note these samples are machine direction samples. An additional four strips 1 inch by 7 inch with the long 7 inch dimension running parallel to the cross direction. All cuts are made using a cutter (JDC-1-10 or JDC-1-12 with safety shield from Thwing-Albert Instrument Co., 10960 Dutton Road, Philadelphia, Pa., 19154). A total of eight samples are produced: four 1 inch by 7 inch strips, 8 plies thick, with the 7 inch dimension running parallel to the machine direction and four 1 inch by 7 inch strips, 8 plies thick, with the 7 inch dimension running parallel to the cross direction.

Each of the four eight ply stacks of machine direction and cross machine direction sample tensile strips are measured in the Thwing-Albert Intelect II Standard Tensile Tester. The four measurements of the 8 ply stacks of machine direction sample tensile strips are summed and divided by four, which is the number of machine direction strips tested. The sum is also divided by eight, which the number of usable units per tensile strip. The calculation is repeated for the cross machine direction measurements.

All results are in units of grams/inch. Appropriate unit conversions may be made to achieve units of grams/cm as reported herein.

(e) Initial Wet Tensile Strength

For the initial wet tensile strength determination, a portion of the test sample is immersed in water for five seconds prior to the tensile strength measurement. The wet tensile strength is determined on one inch wide strips of sample using a Thwing-Albert Intelect II Standard Tensile Tester (Thwing-Albert Instrument Co., 10960 Dutton Rd., Philadelphia, Pa., 19154) and a Finch Wet Strength Device, Catalog Number 731D (Thwing-Albert Instrument Co., 10960 Dutton Rd., Philadelphia, Pa., 19154).

Prior to sample preparation and wet tensile testing, the samples should be cured in a forced draft oven at 105 ± 3 degree Celsius for a period of 5 minutes ± 10 seconds. The samples should be suspended in the oven such that the forced air can circulate between them.

Sample preparation and all aspects of the wet tensile testing should take place within the confines of the constant temperature and humidity room. Two stacks of 5 plies each are made from the cured samples after conditioning. From one of these stacks of 5 plies, four strips are cut 1 inch by 4 inch with the long 4 inch dimension running parallel to the machine direction for machine direction samples. An additional four strips are cut 1 inch by 4 inch with the long 4 inch dimension running parallel to the cross direction for cross direction samples. All cuts are made using a paper cutter (JDC-1-10 or JDC-1-12 with safety shield from Thwing-Albert Instrument Co., 10960 Dutton Road, Philadelphia, Pa., 19154). There are a total of eight samples: four 1 inch by 4 inch strips which are 5 plies thick with the 4 inch dimension running parallel to the machine direction and four 1 inch by 4 inch strips which are 5 plies thick with the 4 inch dimension running parallel to the cross direction.

Each of the four five ply stacks of machine direction and cross machine direction sample tensile strips are measured in the Thwing-Albert Intelect II Standard Tensile Tester. The four measurements of the 5 ply stacks of machine direction sample tensile strips are summed and divided by four, which is the number of machine direction strips tested. The sum is also divided by five, which the number of usable units per tensile strip. The calculation is repeated for the cross machine direction measurements.

All results are in units of grams/inch. Appropriate unit conversions may be made to achieve units of grams/cm as reported herein.

(f) Decayed Wet Tensile (Soaked for 30 minutes)

Same as the Initial Wet Tensile Strength except the samples are allowed to soak in the water for 30 minutes (± 30 seconds) prior to Wet Tensile Strength Testing.

(g) Flexibility

Flexibility as used herein is defined as the slope of the secant of the graph-curve derived from force vs. stretch % data which secant passes through the origin (zero % stretch, zero force) and through the point on the graph-curve where the force per centimeter of width is 20 grams. For example, for a sample which stretches 10% (i.e., 0.1 cm/cm of length) with 20 grams of force per cm of sample width, the slope of the secant through (0%, 0) and (10%, 20) is 2.0 using the formula:

$$Slope = \frac{Y_2 - Y_1}{X_2 - X_1}$$

Total Flexibility as used herein means the geometric mean of the machine-direction flexibility and cross-machine-direction flexibility. Mathematically, this is the square root of the product of the machine-direction flexibility and cross-machine-direction flexibility in grams per cm.

(h) Absorbency

Absorbency herein is defined as the amount (grams) of distilled water at $73 \pm 2$ °F per gram of sample held by the sample after it has been submerged in a water bath for a period of $30 \pm 3$ seconds and then allowed to sit in a horizontal position for $120 \pm 5$ seconds followed by $60 \pm 5$ seconds sitting at a 75° angle (as measured off of horizontal).

A preconditioned sample is cut to a size of 11 inches by 11 inches. The Machine Direction of the sample is marked and the sample is weighed on a torsion balance to $\pm 0.01$ grams and recorded. This is known as the Sample Dry Weight. After weighing the sample, the dry sample rack (further described below) is placed on the balance and the weight is recorded to $\pm 0.01$ grams. This is known as the Rack Dry Weight

The sample is placed on a rack and covered with a rack cover, further described below. The sample, contained by the rack and rack cover, is gently and completely submerged (to a depth of 2 to 3 inches) horizontally in a bath of distilled water at a temperature of $73 \pm 2$ °F for $30 \pm 3$ seconds.

After being submerged for $30 \pm 3$ seconds, the sample is gently raised (horizontally), the rack cover is gently removed, and the sample and rack are allowed to sit for a period of $120 \pm 5$ seconds in order to drain. While the sample is sitting in the horizontal position, water sitting on the rack is gently wiped off without touching the sample.

Following the drying of the rack and the completion of the horizontal sitting period, the rack and sample together are gently raised so that the Machine Direction is at an angle of 75° from horizontal and allowed to sit in this position for a period of $60 \pm 5$ seconds. After this sitting period is completed, the rack and sample are returned to a horizontal position and once again the rack is dried of standing water. The rack and sample are gently placed on the balance and the weight $\pm 0.01$ grams is recorded. This is known as the Sample and Rack Wet Weight.

The absorbency measurement is made and recorded for three (3) machine direction samples and three (3) cross machine direction samples. During the cross machine direction measurement, the cross-machine direction of the sample is placed at an angle of 75° from horizontal.

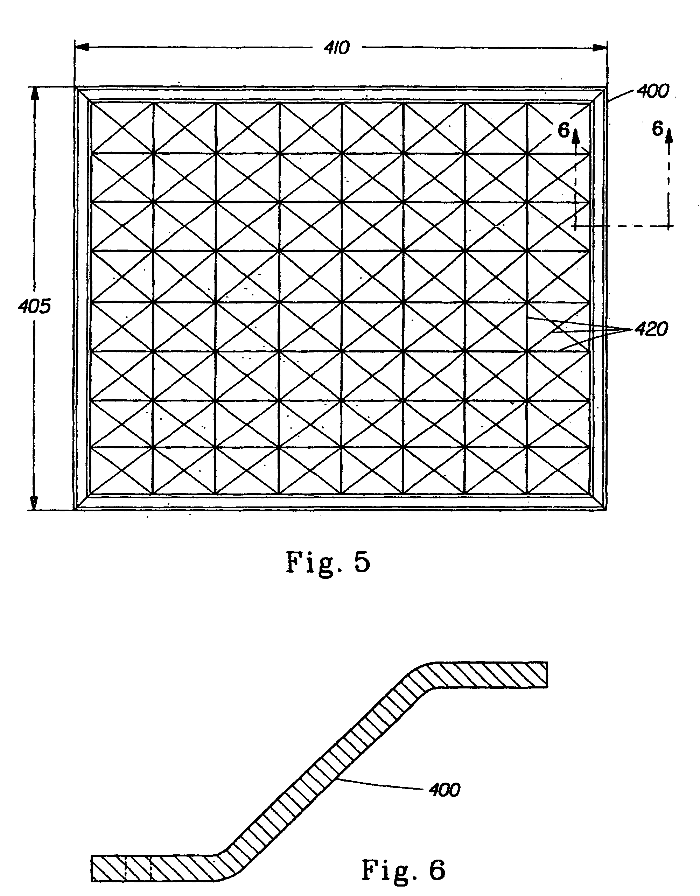

An illustration of the sample rack and cover is shown in Figure 5. Both include frames 400 constructed of 16 GA Aluminum (Teflon Coated after Fabrication) with a cross-section shown in Figure 6. The outside dimensions 405, 410 of the frames 400 are about 13.75 inches by about 16.75 inches. Nylon thread 420 (0.3mm diameter) is tightly strung across the Aluminum frames 400 in a pattern shown in Figure 5. All diagonal threads go over those threads running perpendicular and/or parallel to the frames 400.

For each of the 6 tests, the following calculation is made (all units are grams):

$$\text{Sample Wet Weight} = \text{Sample and Rack Wet Weight} - \text{Rack Dry Weight}$$

$$\text{Absorbency} = \frac{(\text{Sample Wet Weight} - \text{Sample Dry Weight})}{\text{Sample Dry Weight}}$$

The calculation is repeated for each of the 6 measurements and all 6 absorbency numbers are averaged together and reported as $g_{Water}/g_{Dry\ Sample}$ (grams of water / grams of sample dry weight).

(i) SHEAR VISCOSITY

The shear viscosity of the composition is measured using a rotational viscometer (Model DSR 500, manufactured by Rheometrics). A preheated sample composition is loaded into the barrel section of the rheometer, and substantially fills the barrel section (about 60 grams of sample is used). The barrel is held at a test temperature of 90 °C. After the loading, air generally bubbles to the surface and does create problems for the run. For a more viscous samples, compaction prior to running the test may be used to rid the molten sample of entrapped air. The viscometer is programmed to ramp the applied stress from 10 dyne/cm to 5000 dyne/cm. The strain experienced by the sample is measure by a strain gauge. The apparent viscosity of the composition can be derived therefrom. Then log (apparent shear viscosity) is plotted against log (shear rate) and the plot is fitted by the power law $\eta = K \gamma^{n-1}$, wherein K is a material constant, $\gamma$ is the shear rate. The reported shear viscosity of the starch composition herein is an extrapolation to a shear rate of 700 s$^{-1}$ using the power law relation.

(j) EXTENSIONAL VISCOSITY

The extensional viscosity is measured using a capillary rheometer (Model Rheograph 2003, manufactured by Geottfert). The measurements are conducted using an orifice die having a diameter D of 0.5 mm and a length L of 0.25 mm (i.e., L/D = 0.5). The die is attached to the lower end of a barrel, which is held at a test temperature of 90°C. A preheated sample composition is loaded into the barrel section of the rheometer, and substantially fills the barrel section. After the loading, air generally bubbles to the surface and does create problems for the run. For more viscous compositions, compaction prior to running the test may be used to rid the molten sample of entrapped air. A piston is programmed to push the sample from the barrel through the orifice die at a chosen rate. As the sample goes from the barrel through the orifice die, the sample experiences a pressure drop. An apparent viscosity can be obtained from the pressure drop and the flow rate of the sample through the orifice die. Corrections are often applied to the apparent viscosity following procedures generally known in the art. A shear correction factor and Cogswell equation are applied to the calculation of the extensional viscosity. The corrected extensional viscosity at 700 $s^{-1}$ is reported.

It is known that the extensional viscosity can be measured using an orifice die and applying the correction factors, following the method described herein. More details of extensional viscosity measurements are disclosed in S. H. Spielberg et al., The Role Of End-Effects On Measurements Of Extensional Viscoisty In Filament Stretching Rheometers, Journal of Non-Newtonian Fluid Mechanics, Vol. 64, 1996, p. 229-267; Bhattacharya, et al., Uniaxial Extensional Viscoisty During Extrusion Cooking From Entrance Pressure Drop Method, Journal of Food Science, Vol. 59, No. 1, 1994, p. 221-226; both are hereby incorporated by reference. It is also known that the extensional viscosity can be measured using a hyperbolic or semi-hyperbolic die. Detailed disclosure of extensional viscosity measurements using a semi-hyperbolic die is disclosed in U.S. Patent No. 5,357,784, issued October 25, 1994 to Collier, the disclosure of which is incorporated herein by reference.

(k) MOLECULAR WEIGHT AND MOLECULAR WEIGHT DISTRIBUTION

The weight-average molecular weight (Mw) and molecular weight distribution (MWD) of starch are determined by Gel Permeation Chromatography (GPC) using a mixed bed column. Parts of the instrument are as follows:

| Pump | Waters Model 600E |
| --- | --- |
| System controller | Waters Model 600E |
| Autosampler | Waters Model 717 Plus |
| Column | PL gel 20 μm Mixed A column (gel molecular weight ranges from 1,000 to 40,000,000) having a length of 600 mm and an internal diameter of 7.5 mm. |
| Detector | Waters Model 410 Differential Refractometer |
| GPC software | Waters Millenium® software |

The column is calibrated with Dextran standards having molecular weights of 245,000; 350,000; 480,000; 805,000; and 2,285,000. These Dextran calibration standards are available from American Polymer Standards Corp., Mentor, OH. The calibration standards are prepared by dissolving the standards in the mobile phase to make a solution of about 2 mg/ml. The solution sits undisturbed overnight. Then it is gently swirled and filtered through a syringe filter (5 μm Nylon membrane, Spartan-25, available from VWR) using a syringe (5 ml, Norm-Ject, available from VWR).

The starch sample is prepared by first making a mixture of 40wt% starch in tap water, with heat applied until the mixture gelatinizes. Then 1.55 grams of the gelatinized mixture is added to 22 grams of mobile phase to make a 3 mg/ml solution which is prepared by stirring for 5 minutes, placing the mixture in an oven at 105°C for one hour, removing the mixture from the oven, and cooling to room temperature. The solution is filtered using the syringe and syringe filter as described above.

The filtered standard or sample solution is taken up by the autosampler to flush out previous test materials in a 100 μl injection loop and inject the present test material into the column. The column is held at 70°C. The sample eluded from the column is measured against the mobile phase background by a differential refractive index detector held at 50°C and with the sensitivity range set at 64. The mobile phase is DMSO with 0.1% w/v LiBr dissolved therein. The flow rate is set at 1.0ml/min and in the isocratic mode (i.e., the mobile phase is constant during the run). Each standard or sample is run through the GPC three times and the results are averaged.

The average molecular weight of the high polymer is provided by the material suppliers.

(1) THERMAL PROPERTIES

Thermal properties of the present starch compositions are determined using a TA Instruments DSC-2910 which has been calibrated with an indium metal standard, which has an melting temperature (onset) of 156.6°C and a heat of melting of 6.80 calories per gram, as reported in the chemical literature. Standard DSC operating procedure per manufacturer's Operating Manual is used. Due to the volatile evolution (e.g., water vapor) from the starch composition during a DSC measurement, a high volume pan equipped with an o-ring seal is used to prevent the escape of volatiles from the sample pan. The sample and an inert reference (typically an empty pan) are heated at the same rate in a controlled environment. When an actual or pseudo phase change occurs in the sample, the DSC instrument measures the heat flow to or from the sample versus that of the inert reference. The instrument is interfaced with a computer for controlling the test parameters (e.g., the heating/cooling rate), and for collecting, calculating and reporting the data.

The sample is weighed into a pan and enclosed with an o-ring and a cap. A typical sample size is 25-65 milligrams. The enclosed pan is placed in the instrument and the computer is programmed for the thermal measurement as follows:

1. equilibrate at 0°C;
2. hold for 2 minutes at 0°C;
3. heat at 10°C/min to 120°C;
4. hold for 2 minutes at 120°C;
5. cool at 10°C/min to 30°C;
6. equilibrate at ambient temperaure for 24 hours, the sample pan may be removed from the DSC instrument and placed in a controlled environment at 30°C in this duration;
7. return sample pan to the DSC instrument and equilibrate at 0°C;
8. hold for 2 minutes;
9. heat at 10°C/min to 120°C;
10. hold for 2 minutes at 120°C;
11. cool at 10°C/min to 30°C and equilibrate; and
12. remove the used sample.

The computer calculates and reports the thermal analysis result as differential heat flow (ΔH) versus temperature or time. Typically the differential heat flow is normalized and reported on per weight basis (i.e, cal/mg). Where the sample exhibits a pseudo phase transition, such as a glass transition, a differential of the ΔH v. time/temperature plot may be employed to more easily determine a glass transition temperature.

(m) WATER SOLUBILITY

A sample composition is made by mixing the components with heat and stirring until a substantially homogeneous mixture is formed. The melt composition is cast into a thin film by spreading it over a Teflon® sheet and cooling at ambient temperature. The film is then dried completely (i.e., no water in the film/composition) in an oven at 100°C. The dried film is then equilibrated to room temperature. The equilibrated film is ground into small pellets.

To determine the % solids in the sample, 2 to 4 grams of the ground sample is placed in a pre-weighed metal pan and the total weight of pan and sample is recorded. The weighed pan and sample is palced in a 100°C oven for 2 hours., and then taken out and weighed immediately. The % solids is calculated as follows:

$$\% \text{ Solids} = \frac{\text{(dried weight of ground sample \& pan - weight of pan)}}{\text{(first weight of ground sample \& pan - weight of pan)}} \times 100$$

To determine the solubility of the sample composition, weigh 10 grams of ground sample in a 250mL beaker. Add deionized water to make a total weight of 100 grams. Mix the sample and water on a stir plate for 5 minutes. After stirring, pour at least 2mL of stirred sample into a centrifuge tube. Centrifuge 1 hour at 20,000g at 10°C. Take the supernatant of the centrifuged sample and read the refractive index. The % solubility of the sample is calculated as follows:.

$$\% \text{ Soluble Solids} = \frac{\text{(Refractive Index \#)} \times 1000}{\% \text{ Solids}}$$

EXAMPLES

The materials used in the Examples are as follows:

Crystal Gum® is a modified starch having a weight-average molecular weight of 100,000; Nadex® is a modified starch having a weight average molecular weight of 2,000; and Instant-n Oil® is a modified starch having a weight average molecular weight of 800,000; all are available from National Starch and Chemicals Corp., Bridgewater, NJ.

Superfloc® A-130 is a carboxylated polyacrylamide having a weight-average molecular weight of 12,000,000 to 14,000,000 and is available from Cytec Co., Stamford, CT.

Nonionic polyacrylamides PAM-a and PAM-b having a weight-average molecular weight of 15,000,000, and 5,000,000 to 6,000,000, respectively, are available from Scientific Polymer Products, Inc., Ontario, NY.

Polyethyleneimine having a weight-average molecular weight of 750,000 is available from Aldrich Chemical Co., Milwaukee, WI.

Parez® 631 NC is a low molecular weight glyoxylated polyacrylamide, and Parez® 802 is a low molecular weight glyoxylated urea resin, both are available from Cytec Co., Stamford, CT.

Pluronic® F87 is nonionic poloxomer, available form BASF corp., Parsippany, NJ.

Urea, sucrose and glyoxal (in 40% solution in water) are available from Aldrich Chemical Co., Milwaukee, WI.

### EXAMPLE 6

A melt processable composition of the invention is prepared by mixing 45wt% starch (Crystal Gum), 40.5wt% urea, 4.5 wt% sucrose, and 9.8wt% free water, and manually stirring to form a slurry. Polyacrylamide (PAM-a, Mw = 15,000,000) is dissolved in water to form a PAM aqueous solution. An aliquot of the polymer/water solution is added to the slurry. Water in the slurry is then evaporated until the weight percent of polyacrylamide in the final mixture is 0.2wt%.

The composition has a shear viscosity of 0.65 Pa•s and an extensional viscosity of 1863.2 Pa•s, at $700s^{-1}$ and 90°C.

### COMPARATIVE EXAMPLE 6b

A comparative starch composition is prepared according to Example 6 except no polyacrylamide is added to the composition. The composition has a shear viscosity of 1.35 Pa•s and an extensional viscosity of 43.02 Pa•s, at $700s^{-1}$ and 90°C. Example 6 and Comparative Example 6b demonstrate that addition of a small amount of high polymer decreases the shear viscosity slightly and significantly increases the extensional viscosity.

### EXAMPLE 7

A melt processable composition of the invention is prepared by mixing 50wt% starch (Crystal Gum), 30wt% urea, 1.5 wt% sucrose, and 18.5wt% free water, and manually stirring to form a slurry. Polyacrylamide (Superfloc A-130, Mw = 12-14,000,000) is dissolved in water to form a PAM aqueous solution. An aliquot of the polymer/water solution is added to the slurry. Water in the slurry is then evaporated until the weight percent of polyacrylamide in the final mixture is 0.003wt%.

The composition has a shear viscosity of 1.12 Pa•s and an extensional viscosity of 46.0 Pa•s, at $700s^{-1}$ and 90°C.

### COMPARATIVE EXAMPLE 7b

A comparative starch composition is prepared according to Example 7 except no polyacrylamide is added to the composition. The composition has a shear viscosity of 1.23 Pa•s and an extensional viscosity of 0.69 Pa•s, at $704s^{-1}$ and 90°C. Example 7 and Comparative Example 7b demonstrate that addition of a small amount of high polymer decreases the shear viscosity slightly and significantly increases the extensional viscosity.

### EXAMPLE 8

A torque rheometer having a melt blowing die is used to process the composition of Example 6. The torque rheometer is illustrated in Figure 6. The torque rheometer assembly 100 includes a drive unit 110 (Model Rheocord 90 available from Haake GmbH), a barrel 120 partitioned into four temperature zones 122, 124, 126 and 128, a feed port 121, and a melt spinning die assembly 131. Twin screw elements 160 (model TW100, from Haake GmbH) are attached to the drive unit 110 and disposed within the barrel 120. A six inch wide melt blowing die assembly 131 (available from JM Laboratories, Dawsonville, GA) is connected to the end of the barrel via a pump 160. The die assembly has a spinneret plate which has 52 holes per linear inch and a hole diameter of 0.015" (0.0381cm), surrounded by a 0.02" wide air passageway 152, from which a high velocity air stream 151 impinges the extruded filaments just below the spinneret plate. The air stream has the effect of simultaneously blowing the filaments away from the spinneret and attenuating the filaments.

The composition of is prepared (as described in Example 6) by mixing 45wt% starch (Crystal Gum), 0.2wt % polyacrylamide (PAM-a), 40.5wt% urea, 4.5 wt% sucrose, and 9.8wt% water. The mixture is gravity-fed via feed port 121 into a torque rheometer. The torque rheometer and die assembly are set as follows:

| Barrel Temperature | |
|---|---|
| Zone 122 | 70°C |
| Zone 124 | 90°C |
| Zone 126 | 90°C |
| Zone 128 | 90°C |
| Torque | 100 rpm |
| Die Temperature | 126.7°C |
| Air Temperature | 126.7°C |
| Air Pressure | 35 psi |
| Pump | 40 rpm |

The mixture is conveyed from the extruder through the pump into the melt blowing die. The resulting attenuated filaments (or fine fibers) of the invention have fiber diameters ranging from 8 to 40 microns.

Note that the weight percent starch in the melt processable composition includes the weight of starch and the weight of bound water (which is on the average about 8 wt% of the starch). It is to be understood that the as-prepared compositions are used for uniaxial and biaxial extensional processes. However, most of the water is lost during the melt process, and the resulting starch fiber, film or like product contains little or no free water. The resulting product does contain some bound water (possible by absorbing moisture from ambient environment). Therefore, the composition of the resulting product may be more appropriately expressed by its solid components, calculated on a dry solid basis. For example, to calculate, on a dry solid basis, the composition of the fiber made according to Example 8, one would take out the 9.8 wt% free water from the overall composition and the 8wt% bound water from the starch, then normalize the remaining solid content to 100%. Thus, the composition of the fiber of Example 8 calculated on a dry solid basis would be 47.8 wt% starch solid (without bound water), 0.23 wt% polyacrylamide, 46.8 wt% urea and 5.2 wt% sucrose.

EXAMPLE 9

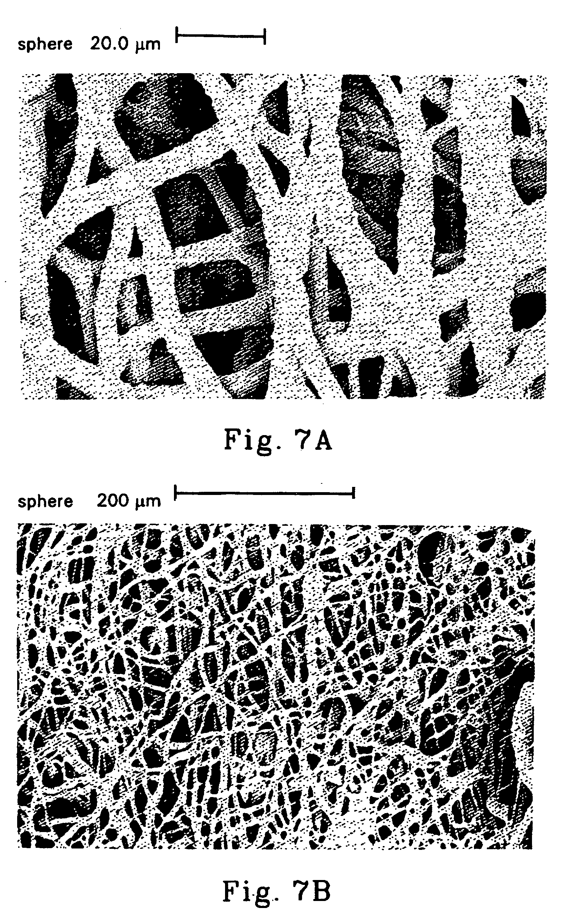

The composition of Example 7 is melt blown into fine fibers of the invention. Figure 7a is the Scanning Electron Micrographs of fine starch fibers made from the composition of Example 7 using the process described in Example 8, shown on a 200 micron scale. Figure 7b is the Scanning Electron Micrographs of the same starch fibers shown on a 20 micron scale. Both figures show that starch fibers of Example 9 have a fairly consistent fiber diameter of about 5 microns.

EXAMPLE 10

Fifteen grams of starch (Crystal Gum, Mw=100,000 ) and fifteen grams of free water are mixed together at 80°C with manual stirring until the mixture becomes substantially homogeneous or gelatinizes. A high polymer (PAM-a, Mw=15,000,000) is dissolved in free water to form a PAM aqueous solution of known concentration. An aliquot of the polymer/water solution is added to the starch/water mixture such that the overall mixture contains 0.006 grams of PAM-a. Then the overall mixture is heated to evaporate water until the weight of the final mixture (starch, PAM-a and water) equals 30 grams. This mixture is subjectively shown to have suitable melt extensibility for drawing fibers.

EXAMPLES 11-13

Mixtures of starch (Crystal Gum), high polymer and water are prepared in the same manner as in Example 5. The final compositions of these mixture are shown below.

| | | Mw | | Ex-11 | Ex-12 | Ex-138 |
|---|---|---|---|---|---|---|
| Starch | Crystal Gum | 100,000 | wt% | 49.99 | 49.99 | 46.92 |
| Polyacrylamide | Superfloc A-130 | 12-14,000,000 | wt% | 0.02 | | |
| | PAM-b | 5-6,000,000 | wt% | | 0.02 | |
| Polyethyleneimine | | 750,000 | wt% | | | 6.17 |
| Water | | | wt% | 49.99 | 49.99 | 46.91 |

These compositions of the invention are subjectively shown to have suitable melt extensibility for drawing fibers.

EXAMPLES 14-16

The following compositions are prepared in the same manner as Example 1.

|  |  | Mw |  | Ex-14 | Ex-15 | Ex-16 |
|---|---|---|---|---|---|---|
| Starch | Crystal Gum | 100,000 | wt% | 41.54 | 20.77 | 20.77 |
|  | Nadex | 2,000 | wt% |  | 20.77 |  |
|  | Instant-n Oil | 800,000 | wt% |  |  | 20.77 |
| Polyacrylamide | PAM-a | 15,000,000 | wt% | 0.08 | 0.08 | 0.08 |
| Urea |  |  | wt% | 6.23 | 6.23 | 6.23 |
| Sucrose |  |  | wt% | 6.23 | 6.23 | 6.23 |
| Parez 631 NC |  |  | wt% | 1.04 | 1.04 | 1.04 |
| Water |  |  | wt% | 44.88 | 44.88 | 44.88 |

These compositions of the invention are expected to have suitable melt extensibility for drawing fibers. And where the water has been adjusted to about pH 2, the resulting fibers are expected to have a water solubility of less than 30%, based on the test method disclosed herein.

EXAMPLE 17

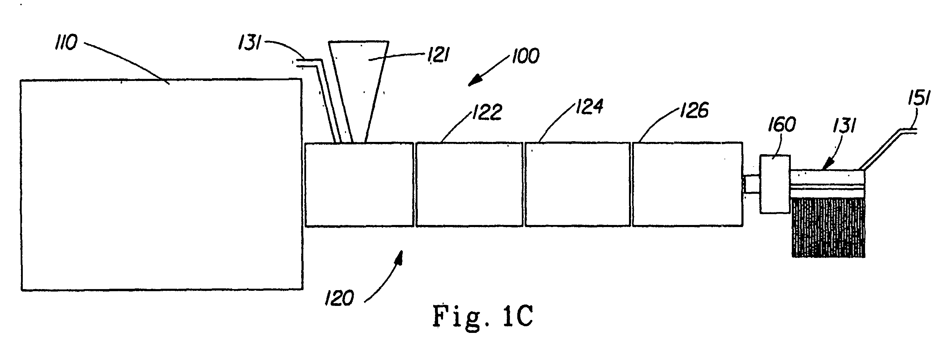

A melt processable composition is prepared by mixing 45wt% starch (Crystal Gum), 0.2wt % polyacrylamide (PAM-a), 40.5wt% urea, 4.5 wt% sucrose, and 9.8wt% water to form a slurry. The composition is melt blown into fine fibers using a torque rheometer as shown in Figure 1c in the manner described in Example 8, except the mixture is meter-fed into the torque rheometer. The torque rheometer and die assembly are set as follows:

| Barrel Temperature |  |
|---|---|
| Zone 122 | 70°C |
| Zone 124 | 90°C |
| Zone 126 | 90°C |
| Zone 128 | 90°C |
| Torque | 140 rpm |
| Feed Rate | 16 gm/min |
| Die Temperature | 137.8°C |
| Air Temperature | 137.8°C |
| Air Pressure | 50 psi |
| Pump | 40 rpm |

The resulting attenuated filaments (or fine fibers) of the invention have fiber diameters ranging from 10 to 30 microns. The fibers are air laid onto a papermaking forming fabric as described in U.S. Patent No. 4,637,859, with the fabrics of U.S. Patent Nos. 5,857,498, 5,672,248, 5,211,815 and 5,098,519, all incorporated herein by reference, also being judged suitable for this purpose.

EXAMPLE 18

The resultant web from the air-laying process of Example 17 is tested for oil absorbency. A drop of a commercially available motor oil (SAE 20 grade, by the Society of Automobile Engineers' designation) is placed on the web and on a commercially available paper towel, respectively, for comparison of oil absorbency. The web shows an improved oil absorbency over that of the commercial paper towel in the following aspects: (1) the web absorbs oil faster than the commercial paper towel, as shown by a shorter residence time on the surface of the web; and (2) after 30 seconds, the web has a spot size of about 1.5 to 2 times larger in diameter than that of the commercial paper towel.

EXAMPLE 19

This example illustrates that the starch composition of the present invention can be made into building materials, e.g., pressed board. A melt processable composition is prepared by mixing 60wt% starch (Crystal Gum), 0.1wt % polyacrylamide (SP2), 2wt% urea, 2 wt% sucrose, 1.5 wt% Parez 631 NC and 34.4 wt% water (adjusted to pH 2 with sulfuric acid) to form a slurry. The slurry is fed in to a torque rheometer (Model Rheocord 90) as illustrated in Figure 1c and operated under the conditions as described in Example 17 above, except a single capillary die (having a 1 mm diameter and a temperature of 90°C) is used instead of a melt spinning die. The extruded strand is dusted with saw dust or wood shavings while still wet and sticky. The dusted strands are compressed together to form a log. The log is dried at 40°C in a forced air oven for two hours to get rid of the residual water from the starch composition. The final product is a log of 47.8wt% saw dust and 52.2 wt% dried starch composition.

EXAMPLE 20

This example illustrates that the present invention can be incorporated into structural materials as reinforcements. Though this example uses fibers made from a composition without high polymers. It is believed that when a composition of the present invention is used, the product would show better or equivalent performances.
A comparative cement sample is prepared as follows: 5 parts of commercially available Quikrete Anchoring cement are mixed with 1.5 part clean tap water until a thick syrup consistency is obtained. Within 5 minutes of mixing, the cement was introduced into cylindrical molds in order to obtain a constant dimension sample for evaluation. Thin wall molds 5" long and 0.23" in inner diameter (i.e., commercially available straws) are filled by driving the pasty cement mixture up from the bottom. This filling method eliminates air inclusion in the finished sample. The samples are allowed to cure for 5 days prior to evaluation. The mold is carefully scored on the outer surface so as not to damage the sample inside, then the mold is peeled away to retrieve the comparative sample (Example 20b).
A melt processable composition is prepared by mixing 45wt% starch (Durabond®, available from National Starch and Chemicals Corp., Bridgewater, NJ), 15 wt% urea, 15 wt% sorbitol, and 25 wt% water to form a slurry. The slurry is fed in to a torque rheometer (Model Rheocord 90) as illustrated in Figure 1c and operated under the condition as described in Example 19 above. The fibers are about 0.02" in diameter and are cut to 1" in length for use herein. The extruded, thin spaghetti-like strands are incorporated into cement as follows: 5 parts of commercially available Quikrete Anchoring cement are mixed with 1.5 part clean tap water and 0.5% (on a dry weight basis) starch fibers. The additional amount of water added herein is required to achieve the comparable consistency as the comparative sample above. The sample molds are filled and the samples (Example 20) are cured and retrieved in the same manner as above.
The samples are subjectively evaluated by bending to failure by hand. Example 20 are subjectively judged to be slightly weaker than the comparative Example 20b. Example 20 has an apparent density of 1.46g/linear inch while comparative Example 20b has an apparent density of 1.48g/linear inch. Therefore, it is demonstrated that Example 20 offers the benefits of light weight and lower cost (on a volume basis).

EXAMPLE 21

This example illustrates that the composition of the present invention can prophetically be made into a controlled water release material when mixed with potting soil. The controlled water release is useful for horticultural and agricultural plants which thrive in a relatively low humidity environment and/or infrequent watering. A melt processable composition is prepared by mixing 50wt% starch (Durabond®, available from National Starch and Chemicals Corp., Bridgewater, NJ), 0.1wt % polyacrylamide (SP2®), 15 wt% urea, 15 wt% sorbitol, 1.5 wt% Parez® and 18.4 wt% water to form a slurry. The slurry is fed in to a torque rheometer (Model Rheocord 90) as illustrated in Figure 1c and operated under the condition as described in Example 19 above. The extruded, thin spaghetti-like strands are allowed to dry before mixing with potting soil. The ratio of starch-based strand to potting soil depends on the requirements of various types of plants. Generally, 10 wt% of starch-based strands in potting soil shows satisfactory water holding/release results.
Examples 22-24 use films made from compositions without the benefit of high polymers. It is believed that when a composition of the present invention is used in each of these examples, the resultant product would show beneficial improvements in properties, e.g., lower caliper, greater flexibility.

EXAMPLE 22

This example illustrates that the compositions of the invention can be made into thin films, using a Werner & Pfleiderer ZSK-30 co-rotating twin-screw extruder with a L/D ratio of 40. The screw configuration consists of four kneading sections and five conveying sections. The extruder barrel consisted of an unheated feed zone followed by seven heated zones, which are designated consecutively as Zones A, B, 1, 2, 3, 4 and 5. The barrel is controlled to the temperature profile summarized below, and the screw speed is set to 150 rpm.

| Zone | A | B | 1 | 2 | 3 | 4 | 5 |
|---|---|---|---|---|---|---|---|
| Temperature °C | 50 | 50 | 50 | 95 | 95 | 95 | 95 |

A melt processable composition is prepared by metering the solid materials into the extruder with a K2V-T20 volumetric feeder (available from K-Tron Inc., Pitman, NJ) and metering the liquid material into Zone 1 of the extruder with a mini pump (available from Milton-Roy, Ivyland, PA). The components are: 44wt% starch (Durabond® A, available from National Starch and Chemicals Corp., Bridgewater, NJ), 18wt% urea, 18 wt% sucrose, and 20 wt% water. The mixture is conveyed from the extruder into a Zenith B-9000 gear pump into a six-inch wide flat film die (available from Killion Extruders, Cedar Grove, NJ) at a flow rate of 33 $cm^3$/min, wherein the gear pump is maintained at 96°C, the film die is maintained at 94°C and the die opening is set at 15 mils. The resultant film is extruded onto a 12-inch wide chill roll (available from Killion Extruders) which is maintained at 37°C. The film is then wound onto a paper core at a speed of 5 fpm. The resultant film is about 1 mil in thickness, slightly tacky to the touch, and exhibits excellent flexibility (i.e., it can be repeatedly bent at a 180 degree angle without breaking or forming a dead fold).

EXAMPLE 23

This example illustrates that the film from Example 23 can be made into a seed carrier for agricultural applications. The seed carrier film made according to this example provides an inexpensive material that can be laid down to cover and seed a large area effectively. The material holds water to facilitate the germination of the seeds, and the material is biodegradable such that no recovery and disposal are required. The film of Example 22 is placed on a single-sided release paper and sprinkled with grass seeds available from Midwestern Supply or other garden supply stores. Another sheet of single-sided release paper is placed on top of the seeds. The assembly is placed between ¼ inch (0.635cm) aluminum plates and inserted into a 6 inch by 6 inch (15.24cm by 15.24cm) Carver hot press that is preheated to 207°C. The assembly is equilibrated under low/contact pressure for one minute, then pressure is increased to a maximum pressure of 6000 pounds. The assembly is held under the maximum pressure for one minute and quickly depressurized. The assembly is taken out of the press and cooled to room temperature. The resulting film composite shows good cohesion between film and seeds such that the film composite can be handled without loss of seeds.

EXAMPLE 24

This example illustrates that the films of Example 22 are fusable such that the films can be made into substantially transparent bags/pouches useful as sealable food storage pouches, shopping bags, garbage bags, grocery bags, and the like. Two pieces of 4 inch by 4 inch (10.16cm by 10.16cm) films are overlaid with a piece of release paper interposed between them. The release paper should be smaller than the films so that at least three edges of the films are in direct contact with each other. A Vertrod impulse sealer (Model 24LAB-SP) is used to seal three sides of the overlaid films. The sealer is set at 50% voltage, 60 psi pressure, a six second dwell time (one second on and 5 seconds off), and for a total sealing time of one minute. The resultant bag shows uniform, welded seals on three sides. The fourth side can optional be sealed to form a completely sealed pouch.

EXAMPLE 25

This example illustrates the water-insoluble starch compositions of the present invention. A composition is prepared by mixing 50 wt% starch (Crystal Gum), a crosslinking additive (the type and the amount of the crosslinking additive are shown in the Table below) and a balance of water which has been adjusted to pH 2 using sulfuric acid. Where glyoxal (in 40% solution in water) is used, there is no need to adjust the water pH. The composition and test sample are prepared according to Test Method for Water Solubility described hereinabove. The results are shown in the Table below:

|  | Solubility: | | |
| --- | --- | --- | --- |
| % Additive | Parez 631 | Glyoxal | Parez 802 |
| 0.00% | 37% | 37% | 37% |
| 0.12% |  | 16% |  |
| 0.20% |  | 10% |  |
| 0.25% | 28% |  | 48% |
| 0.32% |  | 11% |  |
| 0.40% |  | 7% |  |
| 0.50% | 16% |  | 16% |
| 0.75% | 14% |  | 9% |
| 1.00% | 14% |  | 6% |
| 1.50% | 11% |  | 4% |

The disclosures of all patents, patent applications (and any patents which issue thereon, as well as any corresponding published foreign patent applications), and publications mentioned throughout this description are hereby incorporated by reference herein. It is expressly not admitted, however, that any of the documents incorporated by reference herein teach or disclose the present invention.

**Claims**

1. An absorbent, flexible structure comprising pseudo-thermoplastic starch fibers that can be softened by heating in the presence of solvents or softeners to a degrese such that they can be brought into a flowable state, such fibers having a size ranging from 0.01 dtex to 5 dtex.

2. The structure of claim 1, wherein the structure has a geometric mean dry tensile strength ranging from 10 g/cm to 1200 g/cm, an initial geometric mean wet tensile strength ranging from 2 g/cm to 400 g/cm, and a geometric mean decayed wet tensile strength ranging from 0 g/cm to 20 g/cm.

3. The structure of claim 1, wherein the structure has a basis weight ranging from 10 g/m$^2$ to 450 g/m$^2$.

4. The structure of claim 1, further comprising a plasticizer selected from the group consisting of sorbitol, monosaccharides, disaccharides, glycerol, polyvinyl alcohol, and polyethylene glycol, wherein said plasticizer comprises, based on total weight of the structure, from 5 wt % to 70 wt %.

5. The structure of claim 1, further comprising cross-linking agents selected from the group consisting of, polyamide-epichlorohydrin resins, urea-formaldehyde resins, glyoxylated polyacrylamide resins, melamine formaldehyde resins, polyethylenimine resins, Caldas 10 resin, CoBond 1000 resin, wherein said cross-linking agents are present in amounts ranging from 0.1 wt % to 10 wt. %, based on the total weight of the structure.

6. The structure of claim 1, wherein the structure has an absorbency ranging from $1 \frac{g_{Water}}{g_{Dry\,Structure}}$ to 15 $\frac{g_{Water}}{g_{Dry\,Structure}}$.

7. The structure of claim 1, wherein the structure has a total flexibility ranging from 1.0 g/cm to 75 g/cm.

8. A structure comprising pseudo-thermoplastic starch fibers that can be softened by heating in the presence of solvents or softeners to a degrese such that they can be brought into a flowable state, such fibers wherein the fibers have a Tg of at least -30°C and the structure has a geometric mean decayed wet tensile strength ranging from 0 g/cm to 20 g/cm and wherein said fibers have a size ranging from 0.01 dtex to 135 dtex.

9. An absorbent structure comprising one or more plies wherein at least one ply comprises pseudo-thermoplastic starch fibers that can be softened by heating in the presence of solvents or softeners to a degrese such that they can be brought into a flowable state, such fibers having a size ranging from 0.01 dtex to 5 dtex and wherein the at least one ply has a basis weight ranging from 10 g/m$^2$ to 100 g/m$^2$, a GMDT ranging from 40 g/cm to 475 g/cm, an apparent density ranging from 0.04 g/cm$^3$ to 0.12 g/cm$^3$, and an initial GMWT ranging from 2 g/cm to 200 g/cm.

**Patentansprüche**

1. Absorbierende flexible Struktur umfassend pseudo-thermoplastische Stärke-Fasern, die durch Erwärmung in der Gegenwart von Lösungsmitteln oder Weichmachern in einem derartigen Maße weich gemacht werden können, dass sie in einen fließfähigen Zustand gebracht werden können, wobei derartige Fasern eine Größe aufweisen, die von 0,01 dtex bis 5 dtex reicht.

2. Struktur nach Anspruch 1, wobei die Struktur aufweist: eine geometrisch mittlere Trocken-Zugfestigkeit, die von 10 g/cm bis 1200 g/cm reicht, eine anfängliche geometrisch mittlere Naß-Zugfestigkeit, die von 2 g/cm bis 400 g/cm reicht, und eine geometrisch mittlere Zersetzungs-Naß-Zugfestigkeit, die von 0 g/cm bis 20 g/cm reicht.

3. Struktur nach Anspruch 1, wobei die Struktur eine Flächenmasse aufweist, die von 10 g/m$^2$ bis 450 g/m$^2$ reicht.

4. Struktur nach Anspruch 1, ferner umfassend ein Weichmachungsmittel, das gewählt ist aus der Gruppe bestehend aus Sorbit, Monosacchariden, Disacchariden, Glycerol, Polyvinylalkohol und Polyethylenglykol, wobei das Weichmachungsmittel, basierend auf dem Gesamt-Gewicht der Struktur, 5 Gew.-% bis 70 Gew.-% aufweist.

5. Struktur nach Anspruch 1, ferner umfassend Vernetzungsmittel, die gewählt sind aus der Gruppe bestehend aus Polyamid-Epichlorhydrin-Harzen, Harnstoff-Formaldehyd-Harzen, glyoxylierte Polyacrylamid-Harzen, Melamin-Formaldehyd-Harzen, Polyethylenimin-Harzen, Caldas-10-Harz, CoBond-1000-Harz, wobei die Vernetzungsmittel in Mengen vorhanden sind, die von 0,1 Gew.-% bis 10 Gew.-%, basierend auf dem Gesamt-Gewicht der Struktur, reichen.

6. Struktur nach Anspruch 1, wobei die Struktur ein Absorptionsvermögen aufweist, das von 1 g$_{Wasser}$/g$_{trockene Struktur}$ bis 15 g$_{Wasser}$/g$_{trockene Struktur}$ reicht.

7. Struktur nach Anspruch 1, wobei die Struktur eine Gesamt-Flexibilität aufweist, die von 1,0 g/cm bis 75 g/cm reicht.

8. Struktur umfassend pseudo-thermoplastische Stärke-Fasern, die durch Erwärmung in der Gegenwart von Lösungsmitteln oder Weichmachern in einem derartigen Maße weich gemacht werden können, dass sie in einen fließfähigen Zustand gebracht werden können, wobei derartige Fasern eine Tg von mindestens -30°C aufweisen und die Struktur eine geometrisch mittlere Zersetzungs-Naß-Zugfestigkeit aufweist, die von 0 g/cm bis 20 g/cm reicht, und wobei die Fasern eine Größe aufweisen, die von 0,01 dtex bis 135 dtex reicht.

9. Absorbierende Struktur umfassend eine oder mehrere Lagen, wobei mindestens eine Lage pseudo-thermoplastische Stärke-Fasern aufweist, die durch Erwärmung in der Gegenwart von Lösungsmitteln oder Weichmachern in einem derartigen Maße weich gemacht werden können, dass sie in einen fließfähigen Zustand gebracht werden können, wobei derartige Fasern eine Größe aufweisen, die von 0,01 dtex bis 5 dtex reicht, und wobei die mindestens eine Lage aufweist: eine Flächenmasse, die von 10 g/m$^2$ bis 100 g/m$^2$ reicht, eine geometrisch mittlere Trocken-Zugfestigkeit (GMTZ), die von 40 g/cm bis 475 g/cm reicht, eine Rohdichte, die von 0,04 g/cm$^3$ bis 0,12 g/cm$^3$ reicht, und eine anfängliche geometrisch mittlere Naß-Zugfestigkeit (GMNZ), die von 2 g/cm bis 200 g/cm reicht.

**Revendications**

1. Structure absorbante flexible comprenant des fibres d'amidon pseudo-thermoplastiques qui peuvent être ramollies par chauffage en présence de solvants ou d'adoucissants à un degré tel qu'ils peuvent être amenés à un état fluide, ces fibres ayant une taille allant de 0,01 dtex à 5 dtex.

2. Structure selon la revendication 1, dans laquelle la structure présente une résistance à la traction allant de 10 g/cm à 1200 g/cm, une résistance à la traction à voie humide moyenne géométrique initiale allant de 2 g/cm à 400 g/cm et une résistance à la traction humide à dégradation moyenne géométrique allant de 0 g/cm à 20 g/cm.

3. Structure selon la revendication 1, dans laquelle la structure présente un poids de base allant de 10 g/m$^2$ à 450 g/m$^2$.

4. Structure selon la revendication 1, comprenant de plus un agent plastifiant choisi dans le groupe consistant en sorbitol, monosaccharides, disaccharides, glycérol, alcool polyvinylique et polyéthylène glycol, dans lequel ledit agent plastifiant comprend, rapporté au poids total de la structure, de 5 % en poids à 70 % en poids.

5. Structure selon la revendication 1, comprenant de plus des agents réticulants choisis dans le groupe consistant en résines polyamide-épichlorhydrine, résines urée-formaldéhyde, résines polyacrylamide glyoxylé, résines formaldéhyde mélamine, résines polyéthylènimine, résine Caldas 10, résine CoBond, dans laquelle les agents réticulants sont présents dans des quantités allant de 0,1 % en poids à 10 % en poids, rapporté au poids total de la structure.

6. Structure selon la revendication 1, dans laquelle la structure a un pouvoir absorbant allant de 1 $g_{eau}/g_{structure\ sèche}$ à 15 $g_{eau}/g_{structure\ sèche}$.

7. Structure selon la revendication 1, dans laquelle la.structure a une flexibilité totale allant de 1,0 g/cm à 75 g/cm.

8. Structure comprenant des fibres d'amidon pseudo-thermoplastiques qui peuvent être ramollies par chauffage en présence de solvants ou d'agents adoucissants jusqu'à des degrés tels qu'ils peuvent être amenés à un état fluide, tel que des fibres, dans lesquelles les fibres ont un Tg d'au moins -30°C et la structure a une résistance à la traction humide en dégradation moyenne géométrique allant de 0 g/cm à 20 g/cm, et dans lequel lesdites fibres ont une taille allant de 0,01 dtex à 135 dtex.

9. Structure absorbante comprenant un ou plusieurs plis dans lesquels au moins un pli comprend des fibres d'amidon pseudo-thermoplastiques qui peuvent être radoucies par chauffage en présence de solvants ou d'agents adoucissants à un degré tel qu'ils peuvent être amenés à un état fluide, ces fibres ayant une taille allant de 0,01 dtex à 5 dtex, et dans lequel au moins un pli a un poids de base allant de 10 g/m$^2$ à 100 g/m$^2$, un GMDT allant de 40 g/cm à 475 g/cm, une densité apparente allant de 0,04 g/cm$^3$ à 0,12 g/cm$^3$ et un GMWT allant de 2 g/cm à 200 g/cm.

Fig. 1A

Fig. 1B

Fig. 1C

Fig. 2A

Fig. 2B

Fig. 3A

Zone 5    Zone 4    Zone 3    Zone 2    Zone 1

Fig. 3B

EP 1 035 239 B1

Extruder

10

12

F

22

Air
Supply

26

20

24

30

Fig. 4

Fig. 5

Fig. 6

sphere   20.0 μm  ├────────┤

Fig. 7A

sphere   200 μm  ├──────────┤

Fig. 7B